# EUROPEAN PATENT APPLICATION

(11) **EP 2 781 522 A1**
(43) Date of publication of application: **24.09.2014**
(21) Application number: 12849626.2
(22) Date of filing: 08.11.2012
(51) Int. Cl.: C07F 7/18, B01J 31/22, C07C 67/333, C07C 69/757, C07C 237/24, C07D 263/14, C07D 263/16, C07B 53/00, C07B 61/00

(54) **OPTICALLY ACTIVE BISOXAZOLINE COMPOUND, ASYMMETRIC CATALYST, AND METHOD FOR PRODUCING OPTICALLY ACTIVE CYCLOPROPANE COMPOUND USING SAID CATALYST**

(30) Priority: 14.11.2011 JP 2011248357
(71) Applicant: Sumitomo Chemical Company, Limited, Tokyo 104-8260 (JP)
(72) Inventor: MATSUMOTO, Tsutomu, Osaka-shi Osaka 554-8558 (JP); MASUMOTO, Katsuhisa, Ibaraki-shi Osaka 567-0864 (JP); TANAKA, Akio, Osaka-shi Osaka 554-8558 (JP)
(74) Representative: Vossius & Partner
(86) International application number: PCT/JP2012/079592
(87) International publication number: WO 2013/073596

(57) **Abstract**

An optically active bisoxazoline compound represented by formula (1): 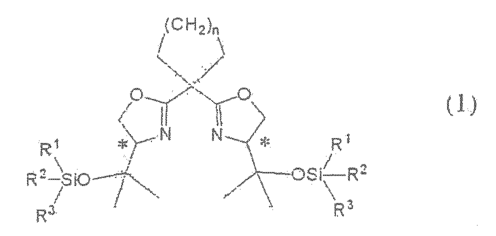 wherein R¹, R² and R³ each independently represent an alkyl group having 1 to 8 carbon atoms, a phenyl group or a benzyl group, n represents an integer of 0 to 3 and * represents an asymmetric center.

## Description

### Technical Field

The present invention relates to an optically active bisoxazoline compound, an asymmetric catalyst, and a process for producing an optically active cyclopropane compound using the same.

### Background Art

As a process for producing an optically active cyclopropane compound, there is known a process of reacting a prochiral olefin with a diazoacetate ester in the presence of an asymmetric catalyst obtained by mixing a monovalent or divalent copper compound and a compcund represented by the following formula (Patent documents 1, 2 and 3).

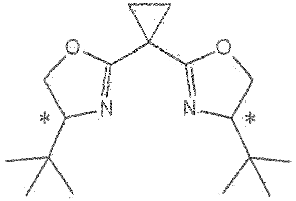

### Prior Technological Documents

### Patent Documents

Patent document 1: US-Patent No. 7709651
Patent document 2: US-Patent No. 7705165
Patent document 3: US-Patent No. 7671210

### Summary of the Invention

### Problem to be Solved by the Invention

In the conventional process, selectivity of a trans isomer in the resulting optically active cyclopropane compound is not necessarily sufficiently satisfactory in some cases.

### Means for Solving the Problem

The present invention includes the following inventions.
[1] An optically active bisoxazoline compound represented by formula (1): 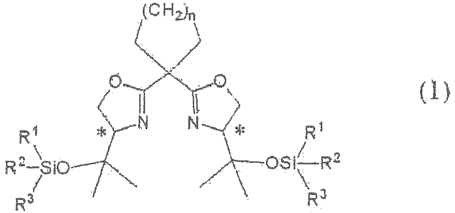 wherein R¹, R² and R³ each independently represent an alkyl group having 1 to 8 carbon atoms, a phenyl group or a benzyl group, n represents an integer of 0 to 3 and * represents an asymmetric center.
[2] The compound according to [1], wherein n is 0.
[3] The compound according to [1] or [2], wherein R¹, R² and R³ each independently represent an ethyl group or an isopropyl group.
[4] The compound according to any one of [1] to [3], wherein R¹, R² and R³ are identical to each other.
[5] The compound according to [1] or [2], wherein R¹ is a thexyl group and R² and R³ are methyl groups.
[6] An asymmetric catalyst obtained by mixing the following (A) and (B):
   (A) a monovalent or divalent copper compound
   (B) the compound according to any one of [1] to [5].
[7] The asymmetric catalyst according to [6], obtained by mixing the (A) and the (B) and the following (C) or (D):
   (C) a boron compound represented by formula (2): 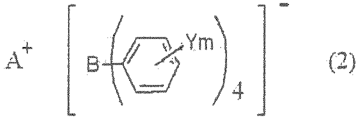 wherein A⁺ represents a lithium ion, a sodium ion, a potassium ion, a silver (I) ion or a trityl cation, Y represents a fluorine atom or a fluorinated alkyl group having 1 to 8 carbon atoms, m represents an integer of 1 to 5, and when m is 2 or more, the plurality of Y are the same or different
   (D) a fluorine compound represented by formula (3):

      D⁺ (MF₆)⁻ (3)

      wherein D⁺ represents a lithium ion, a sodium ion, a potassium ion, a silver(I) ion or a trityl cation and M represents a phosphorus atom, an arsenic atom or an antimony atom.
[8] The asymmetric catalyst according to [6], obtained by mixing the (A), the (B) and the following (C):
   (C) a boron compound represented by formula (2): 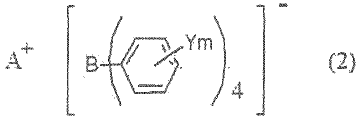 wherein A⁺ represents a trityl cation, Y represents a fluorine atom or a fluorinated alkyl group having 1 to 6 carbon atoms, m represents an integer of 1 to 5, and when m is 2 or more, the plurality of Y are the same or different.
[9] A process for producing an optically active cyclopropane compound represented by formula (6): 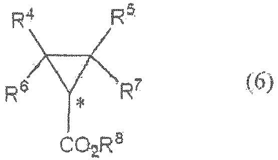 wherein R⁴, R⁵, R⁶, R⁷ and R⁸ are as defined below and * represents an asymmetric center,
   the process comprising a step of reacting a prochiral olefin represented by formula (4): 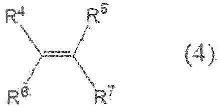 wherein R⁴, R⁵, R⁶ and R⁷ each independently represent a hydrogen atom, a halogen atom, an alkyl group having 1 to 6 carbon atoms and optionally having a substituent, an alkenyl group having 2 to 6 carbon atoms and optionally having a substituent, an aryl group having 6 to 10 carbon atoms and optionally having a substituent, an aralkyl group having 7 to 16 carbon atoms and optionally having a substituent or an alkoxycarbonyl group having 2 to 7 carbon atoms and optionally having a substituent, and R⁵ and R⁷ represent groups different from each other when R⁴ and R⁶ represent the same group,
   with a diazoacetate ester represented by formula (5):

   N₂CHCO₂R⁸ (5)

   wherein R⁸ represents an alkyl group having 1 to 6 carbon atoms, in the presence or the asymmetric catalyst according to any one of [6] to [8].
[10] The process according to [9], wherein R⁴ is a hydrogen atom or a fluorine atom, R⁵ is a methyl group, R⁶ is an acyloxymethyl group, an alkoxycarbonyloxymethyl group or an aralkyloxymethyl group and R⁷ is a methyl group.
[11] The process according to [10], wherein R⁶ is an acetoxymethyl group or a benzyloxymethyl group.
[12] A process for producing an optically active bisoxazoline compound represented by formula (1): 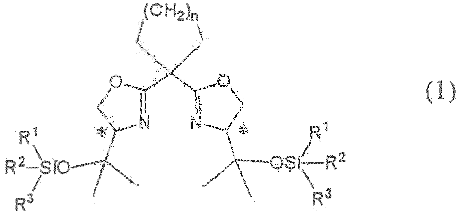 wherein R¹, R² and R³ each independently represent an alkyl group having 1 to 8 carbon atoms, a phenyl group or a benzyl group, n represents an integer of 0 to 3 and * represents an asymmetric center,
   comprising the following first step, second step, third step and fourth step:
   <First step>
      a step of reacting an optically active serine ester represented by formula (7): 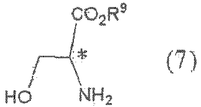 wherein R⁹ represents an alkyl group having 1 to 6 carbon atoms, a phenyl group or a benzyl group, and * represents an asymmetric center,
      or a salt thereof with a cycloalkanedicarboxylic acid derivative represented by formula (8): 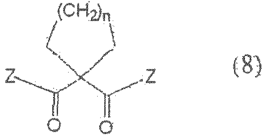 wherein Z represents a halogen atom or an alkoxy group having 1 to 6 carbon atoms, and n represents an integer of 0 to 3, to give an optically active diamide compound represented by formula (9): 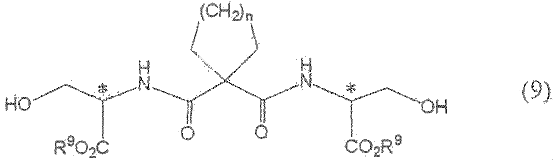 wherein R⁹, n and * are as defined above,
   <Second step>
      a step of reacting the optically active diamide compound represented by formula (9) with a (methoxycarbonylsulfamoyl)triethylammonium hydroxide intramolecular salt to give an optically active bisoxazoline ester compound represented by formula (11): 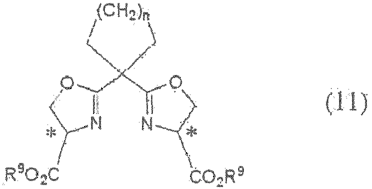 wherein R⁹, n and * are as defined above,
   <Third step>
      a step of reacting the optically active bisoxazoline ester compound represented by formula (11) with a Grignard reagent represented by formula (12):

      CH₃MgQ (12)

      wherein Q represents a halogen atom,
      to give an optically active bisoxazoline alcohol compound represented by formula (13): 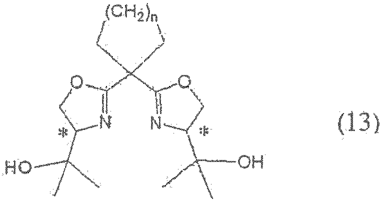 wherein, n and * are as defined above,
   <Fourth step>
      a step of reacting the optically active bisoxazoline alcchol compound represented by formula (13) with a silicon compound represented by formula (14): 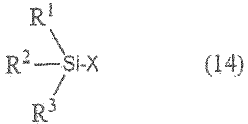 wherein R¹, R² and R³ are as defined above, and X represents a halogen atom,
      to give the optically active bisoxazoline compound represented by formula (1).
[13] An optically active diamide compound represented by formula (9): 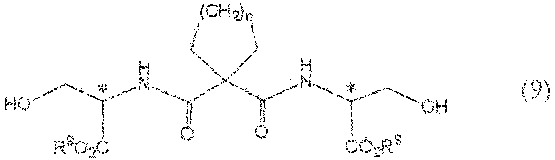 wherein R⁹ represents an alkyl group having 1 to 6 carbon atoms, a phenyl group or a benzyl group, n represents an integer of 0 tc 3 and * represents an asymmetric center.
[14] An optically active bisoxazoline ester compound represented by formula (11): 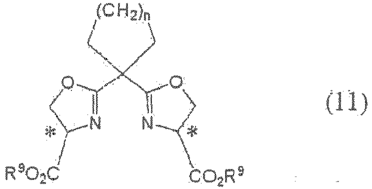 wherein R⁹ represents an alkyl group having 1 to 6 carbon atoms, a phenyl group or a benzyl group, n represents an integer of 0 to 3 and * represents an asymmetric center.
[15] An optically active bisoxazoline alcohol compound represented by formula (13): 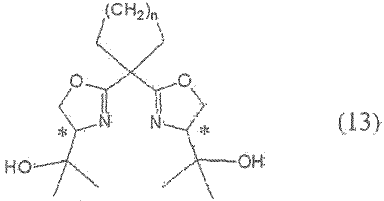 wherein n represents an integer of 0 to 3 and * represents an asymmetric center.

### Effect of the Invention

According to the process of the present invention, selectivity of a trans isomer in the resulting optically active cyclopropane compound is improved.

### Modes for Carrying Out the Invention

### <<Compound (1)>>

An optically active bisoxazoline compound represented by formula (1): 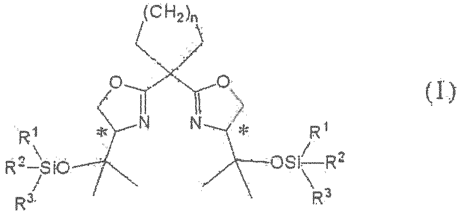 wherein R¹, R² and R³ each independently represent an alkyl group having 1 to 8 carbon atoms, a phenyl group or a benzyl group, n represents an integer of 0 to 3 and * represents an asymmetric center,
(hereinafter, referred to as compound (1) in some cases) will be explained.

Examples of the alkyl group having 1 to 8 carbon atoms represented by R¹, R² and R³ include linear or branched alkyl groups such as a methyl group, an ethyl group, a n-propyl group, an isopropyl group, a n-butyl group, an isobutyl group, a t-butyl group, a n-pentyl group, a n-hexyl group, a cyclohexyl group, a thexyl group, a n-heptyl group and a n-octyl group.

It is preferable that R¹, R² and R³ are each independently an alkyl group having 1 to 8 carbon atoms, and it is more preferable that R¹, R² and R³ are an ethyl group or an isopropyl group; are identical to each other; or R¹ is a thexyl group and R² and R³ are methyl groups.

n is an integer of 0 to 3. A carbon atom to which two bisoxazoline rings are bonded links to -CH₂-(CH₂)ₙ-CH₂- to form an aliphatic ring having 3 to 6 carbon atoms. Examples of the aliphatic ring having 3 to 6 carbon atoms include a cyclopropane ring (n=0), a cyclobutane ring (n=1), a cyclopentane ring (n=2) and a cyclohexane ring (n=3), preferably a cyclopropane ring (n=0).

Examples of the optically active bisoxazoline compound (1) include
1,1-bis[2-[(4R)-4-[2-(trimethylsilyl)oxy-2-propyl]oxazoline ]]cyclopropane,
1,1-bis[2-[(4R)-4-[2-(isopropyldimethylsilyl)oxy-2-propyl]o xazoline]]cyclopropane,
1,1-bis[2-[(4R)-4-[2-(n-butyldimethylsilyl)oxy-2-propyl]oxa zolinel]cyclopropane,
1,1-bis[2-[(4R)-4-[2-(tert-butyldimethylsilyl)oxy-2-propyl] oxazoline]]cyclopropane,
1,1-bis[2-[(4R)-4-[2-(thexyldimethylsilyl)oxy-2-propyl]oxaz oline]]cyclopropane,
1,1-bis[2-[(4R)-4-[2-(cyclohexyldimethylsilyl)oxy-2-propyl] oxazoline]]cyclopropane,
1,1-bis[2-[(4R)-4-[2-(n-octyldimethylsilyl)oxy-2-propyl]oxa zoline]]cyclopropane,
1,1-bis[2-[(4R)-4-[2-(triethylsilyl)oxy-2-propyl]oxazoline] ]cyclopropane,
1,1-bis[2-[(4R)-4-[2-(isopropyldiethylsilyl)oxy-2-propyl]ox azoline]]cyclopropane,
1,1-bis[2-[(4R)-[2-(tri-n-propylsilyl)oxy-2-propyl]oxazolin e]]cyclopropane,
1,1-bis[2-[(4R)-4-[2-(triisopropylsilyl)oxy-2-propyl]oxazol ine]]cyclopropane,
1,1-bis[2-[(4R)-1-[2-(n-octyldiisopropylsilyl)oxy-2-propyl] oxazoline]]cyclopropane,
1,1-bis[2-[(4R)-4-[2-(tri-n-butylsilyl)oxy-2-propyl]oxazoli ne]]cyclopropane,
1,1-bis[2-[(4R)-4-[2-(triisobutylsilyl)oxy-2-propyl]oxazoli ne]]cyclopropane,
1,1-bis[2-[(4R)-4-[2-(phenyldimethylsilyl)oxy-2-propyl]oxaz oline]]cyclopropane,
1,1-bis[2-[(4R)-4-[2-(pentafluorophenyldimethylsilyl)oxy-2-propyl]oxazoline]]cyclopropane,
1,1-bis[2-[(4R)-4-[2-(methyldiphenylsilyl)oxy-2-propyl]oxaz oline]]cyclopropane,
1,1-bis[2-[(4R)-4-[2-(tert-butyldiphenylsilyl)oxy-2-propyl] oxazoline]]cyclopropane,
1,1-bis[2-[(4R)-4-[2-(triphenylsilyl)oxy-2-propyl]oxazoline ]]cyclopropane,
1,1-bis[2-[(4R)-4-[2-(benzyldimethylsilyl)oxy-2-propyl]oxaz oline]]cyclopropane,
1,1-bis[2-[(4R)-4-[2-(tribenzylsilyl)oxy-2-propyl]oxazoline ]]cyclopropane,
1,1-bis[2-[(4R)-4-[2-(trimethylsilyl)oxy-2-propyl]oxazoline ]]cyclobutane,
1,1-bis[2-[(4R)-4-[2-(isopropyldimethylsilyl)oxy-2-propyl]o xazoline]]cyclobutane,
1,1-bis[2-[(4R)-4-[2-(n-butyldimethylsilyl)oxy-2-propyl]oxa zoline]]cyclobutane,
1,1-bis[2-[(4R)-4-[2-(tert-butyldimethylsilyl)oxy-2-propyl] oxazoline]]cyclobutane,
1,1-bis[2-[(4R)-4-[2-(thexyldimethylsilyl)oxy-2-propyl]oxaz oline]]cyclobutane,
1,1-bis[2-[(4R)-4-[2-(cyclohexyldimethylsilyl)oxy-2-propyl] oxazoline]]cyclobutane,
1,1-bis[2-[(4R)-4-[2-(n-octyldimethylsilyl)oxy-2-propyl]oxa zoline]]cyclobutane,
1,1-bis[2-[(4R)-4-[2-(triethylsilyl)oxy-2-propyl]oxazoline] ]cyclobutane,
1,1-bis[2-[(4R)-4-[2-(isopropyldiethylsilyl)oxy-2-propyl]ox azoline]]cyclobutane,
1,1-bis[2-[(4R)-4-[2-(tri-n-propylsilyl)oxy-2-propyl]oxazol ine]]cyclobutane,
1,1-bis[2-[(4R)-4-[2-(triisopropylsilyl)oxy-2-propyl]oxazol ine]]cyclobutane,
1,1-bis[2-[(4R)-4-[2-(n-octyldiisopropylsilyl)oxy-2-propyl] oxazoline]]cyclobutane,
1,1-bis[2-[(4R)-4-[2-(tri-n-butylsilyl)oxy-2-propyl]oxazoli ne]]cyclobutane,
1,1-bis[2-[(4R)-4-[2-(triisobutylsilyl)oxy-2-propyl]oxazoli ne]]cyclobutane,
1,1-bis[2-[(4R)-4-[2-(phenyldimethylsilyl)oxy-2-propyl]oxaz oline]]cyclobutane,
1,1-bis[2-[(4R)-4-[2-(pentafluorophenyldimethylsilyl)oxy-2-propyl]oxazoline]]cyclobutane,
1,1-bis[2-[(4R)-4-[2-(methyldiphenylsilyl)oxy-2-propyl]oxaz oline]]cyclobutane,
1,1-bis[2-[(4R)-4-[2-(tert-butyldiphenylsilyl)oxy-2-propyl] oxazoline]]cyclobutane,
1,1-bis[2-[(4R)-4-[2-(triphenylsilyl)oxy-2-propyl]oxazoline ]]cyclobutane,
1,1-bis[2-[(4R)-4-[2-(benzyldimethylsilyl)oxy-2-propyl]oxaz oline]]cyclobutane,
1,1-bis[2-[(4R)-4-[2-(tribenzylsilyl)oxy-2-propyl]oxazoline ]]cyclobutane,
1,1-bis[2-[(4R)-4-[2-(trimethylsilyl)oxy-2-propyl]oxazoline ]]cyclopentane,
1,1-bis[2-[(4R)-4-[2-(isopropyldimethylsilyl)oxy-2-propyl]o xazoline]]cyclopentane,
1,1-bis[2-[(4R)-4-[2-(n-butyldimethylsilyl)oxy-2-propyl]oxa zoline]]cyclopentane,
1,1-bis[2-[(4R)-4-[2-(tert-butyldimethylsilyl)oxy-2-propyl] oxazoline]]cyclopentane,
1,1-bis[2-[(4R)-4-[2-(thexyldimethylsilyl)oxy-2-propyl]oxaz oline]]cyclopentane,
1,1-bis[2-[(4R)-4-[2-(cyclohexyldimethylsilyl)oxy-2-propyl] oxazoline]]cyclopentane,
1,1-bis[2-[(4R)-4-[2-(n-octyldimethylsilyl)oxy-2-propyl]oxa zoline]]cyclopentane,
1,1-bis[2-[(4R)-4-[2-(triethylsilyl)oxy-2-propyl]oxazoline] ]cyclopentane,
1,1-bis[2-[(4R)-4-[2-(isopropyldiethylsilyl)oxy-2-propyl]ox azoline]]cyclcpentane,
1,1-bis[2-[(4R)-4-[2-(tri-n-propylsilyl)oxy-2-propyl]oxazol ine]]cyclopentane,
1,1-bis[2-[(4R)-4-[2-(triisopropylsilyl)oxy-2-propyl]oxazol ine]]cyclopentane,
1,1-bis[2-[(4R)-4-[2-(n-octyldiisopropylsilyl)oxy-2-propyl] oxazoline]]cyclopentane,
1,1-bis[2-[(4R)-4-[2-(tri-n-butylsilyl)oxy-2-propyl]oxazoli ne]]cyclopentane,
1,1-bis[2-[(4R)-4-[2-(triisobutylsilyl)oxy-2-propyl]oxazoli ne]]cyclopentane,
1,1-bis[2-[(4R)-4-[2-(phenyldimethylsilyl)oxy-2-propyl]oxaz oline]]cyclopentane,
1,1-bis[2-[(4R)-4-[2-(pentafluorophenyldimethylsilyl)oxy-2-propyl]oxazoline]]cyclopentane,
1,1-bis[2-[(4R)-4-[2-(methyldiphenylsilyl)oxy-2-propyl]oxaz oline]]cyclopentane,
1,1-bis[2-[(4R)-4-[2-(tert-butyldiphenylsilyl)oxy-2-propyl] oxazoline]]cyclopentane,
1,1-bis[2-[(4R)-4-[2-(triphenylsilyl)oxy-2-propyl]oxazoline ]]cyclopentane,
1,1-bis[2-[(4R)-4-[2-(benzyldimethylsilyl)oxy-2-propyl]oxaz oline]]cyclopentane,
1,1-bis[2-[(4R)-4-[2-(tribenzylsilyl)oxy-2-propyl]oxazoline ]]cyclopentane
1,1-bis[2-[(4R)-4-[2-(trimethylsilyl)oxy-2-propyl]oxazoline ]]cyclohexane,
1,1-bis[2-[(4R)-4-[2-(isopropyldimethylsilyl)oxy-2-propyl]o xazoline]]cyclohexane,
1,1-bis[2-[(4R)-4-[2-(n-butyldimethylsilyl)oxy-2-propyl]oxa zoline]]cyclohexane,
1,1-bis[2-[(4R)-4-[2-(tert-butyldimethylsilyl)oxy-2-propyl] oxazoline]]cyclohexane,
1,1-bis[2-[(4R)-4-[2-(thexyldimethylsilyl)oxy-2-propyl]oxaz oline]]cyclohexane,
1,1-bis[2-[(4R)-4-[2-(cyclohexyldimethylsilyl)oxy-2-propyl] oxazoline]]cyclohexane,
1,1-bis[2-[(4R)-4-[2-(n-octyldimethylsilyl)oxy-2-propyl]oxa zoline]]cyclohexane,
1,1-bis[2-[(4R)-4-[2-(triethylsilyl)oxy-2-propyl]oxazoline] ]cyclohexane,
1,1-bis[2-[(4R)-4-[2-(isopropyldiethylsilyl)oxy-2-propyl]ox azoline]]cyclohexane,
1,1-bis[2-[(4R)-4-[2-(tri-n-propylsilyl)oxy-2-propyl]oxazol ine]]cyclohexane,
1,1-bis[2-[(4R)-4-[2-(triisopropylsilyl)oxy-2-propyl]oxazol ine]]cyclohexane,
1,1-bis[2-[(4R)-4-[2-(n-octyldiisopropylsilyl)oxy-2-propyl] oxazoline]]cyclohexane,
1,1-bis[2-[(4R)-4-[2-(tri-n-butylsilyl)oxy-2-propyl]oxazoli ne]]cyclohexane,
1,1-bis[2-[(4R)-4-[2-(triisobutylsilyl)oxy-2-propyl]oxazoli ne]]cyclohexane,
1,1-bis[2-[(4R)-4-[2-(phenyldimethylsilyl)oxy-2-propyl]oxaz oline]]cyclohexane,
1,1-bis[2-[(4R)-4-[2-(pentafluorophenyldimethylsilyl)oxy-2-propyl]oxazoline]]cyclohexane,
1,1-bis[2-[(4R)-4-[2-(methyldiphenylsilyl)oxy-2-propyl]oxaz oline]]cyclohexane,
1,1-bis[2-[(4R)-4-[2-(tert-butyldiphenylsilyl)oxy-2-propyl] oxazoline]]cyclohexane,
1,1-bis[2-[(4R)-4-[2-(triphenylsilyl)oxy-2-propyl]oxazoline ]]cyclohexane,
1,1-bis[2-[(4R)-4-[2-(benzyldimethylsilyl)oxy-2-propyl]oxaz oline]]cyclohexane, and
1,1-bis[2-[(4R)-4-[2-(tribenzylsilyl)oxy-2-propyl]oxazoline ]]cyclohexane;
compounds obtained by replacing the steric configuration (4R) at 4-position of an oxazoline ring of each of the above-described compounds with (4S), that is, 1,1-bis[2-[(4S)-4-[2-(triisopropylsilyl)oxy-2-propyl]oxazol ine]]cyclopropane and the like; and
compounds in which the steric configuration of one of two oxazoline skeletons of each of the above-described compounds is (4R) and the steric configuration of the other is (4S), that is, 1-[2-[(4R)-4-[2-(triisopropylsilyl)oxy-2-propyl]oxazoline]] -1-[2-[(4S)-4-[2-(triisopropylsilyl)oxy-2-propyl]oxazoline] ]cyclopropane.

### <<Process for producing compound (1) and intermediate thereof>>

The process for producing a compound (1) includes the following first step, second step, third step and fourth step. In the process for producing a compound (1), the first step, the second step, the third step and the fourth step are preferably carried out in this order. The process for producing a compound (1) may include a step of isolating or purifying the product obtained in each of the steps.

### <First step>

The first step is a step of reacting an optically active serine ester represented by formula (7): 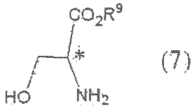 wherein R⁹ represents an alkyl group having 1 to 6 carbon atoms, a phenyl group or a benzyl group and * represents an asymmetric center,
or a salt thereof (hereinafter, referred to as compound (7) in some cases) with a cycloalkanedicarboxylic acid derivative represented by formula (8): 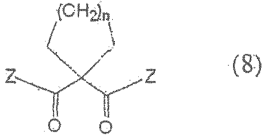 wherein Z represents a halogen atom or an alkoxy group having 1 to 6 carbon atoms and n represents an integer of 0 to 3,
(hereinafter, referred to as compound (8) in some cases) to give an optically active diamide compound represented by formula (9): 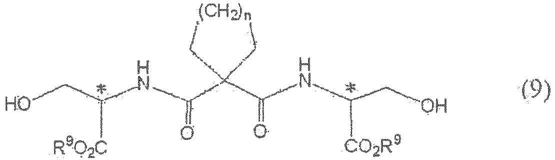 wherein R⁹, n and * are as defined above,
(hereinafter, referred to as compound (9) in some cases).

Examples of the alkyl group having 1 to 6 carbon atoms represented by R⁹ include linear or branched alkyl groups such as a methyl group, an ethyl group, an-propyl group, an isopropyl group, a n-butyl group, an isobutyl group, a tert-butyl group, a n-pentyl group, a n-hexyl group, a cyclohexyl group and a thexyl group.

Examples of the compound (7) include D-serine methyl ester, D-serine ethyl ester, D-serine n-propyl ester, D-serine isopropyl ester, D-serine n-butyl ester, D-serine isobutyl ester, D-serine tert-butyl ester, D-serine n-pentyl ester, D-serine n-hexyl ester, D-serine cyclohexyl ester, D-serine thexyl ester, D-serine phenyl ester, D-serine benzyl ester, salts of these compounds such as a hydrochloride, a hydrobromide, a hydroiodide, a methanesulfonate, a benzenesulfonate and a p-toluenesulfonate thereof, and compounds obtained by replacing the steric configuration D of the above-described compound with L.

As the compound (7), commercially available compounds, and those produced from optically active serine by processes described in Tetrahedron Letters, vol. 45, pp. 9649-9652 (2004), Tetrahedron: Asymmetry, vol. 22, pp. 580-586 (2011), European Journal of Organic Chemistry, pp. 5386-5394 (2007) and the like can be used.

Examples of the halogen atom represented by Z include a chlorine atom and a bromine atom, and examples of the alkoxy group having 1 to 6 carbon atoms include linear or branched alkoxy groups such as a methoxy group, an ethoxy group, a n-propoxy group, an isopropoxy group, a n-butoxy group, an isobutoxy group, a t-butoxy group, a n-pentyloxy group, a n-hexyloxy group, a cyclohexyloxy group and a thexyloxy group.

Examples of the compound (8) include 1,1-cyclopropanedicarboxylic acid dichloride, 1,1-cyclopropanedicarboxylic acid dibromide, 1,1-cyclobutanedicarboxylic acid dichloride, 1,1-cyclobutanedicarboxylic acid dibromide, 1,1-cyclopentanedicarboxylic acid dichloride, 1,1-cyclopentanedicarboxylic acid dibromide, 1,1-cyclohexanedicarboxylic acid dichloride, 1,1-cyclohexanedicarboxylic acid dibromide, 1,1-cyclopropanedicarboxylic acid dimethyl ester, 1,1-cyclopropanedicarboxylic acid diethyl ester, 1,1-cyclopropanedicarboxylic acid di-n-propyl ester, 1,1-cyclopropanedicarboxylic acid diisopropyl ester, 1,1-cyclopropanedicarboxylic acid di-n-butyl ester, 1,1-cyclopropanedicarboxylic acid diisobutyl ester, 1,1-cyclopropanedicarboxylic acid di-n-pentyl ester, 1,1-cyclopropanedicarboxylic acid di-n-hexyl ester, 1,1-cyclopropanedicarboxylic acid dicyclohexyl ester, 1,1-cyclobutanedicarboxylic acid dimethyl ester, 1,1-cyclobutanedicarboxylic acid diethyl ester, 1,1-cyclobutanedicarboxylic acid di-n-propyl ester, 1,1-cyclobutanedicarboxylic acid diisopropyl ester, 1,1-cyclobutanedicarboxylic acid di-n-butyl ester, 1,1-cyclobutanedicarboxylic acid diisobutyl ester, 1,1-cyclobutanedicarboxylic acid di-n-pentyl ester, 1,1-cyclobutanedicarboxylic acid di-n-hexyl ester, 1,1-cyclobutanedicarboxylic acid dicyclohexyl ester, 1,1-cyclopentanedicarboxylic acid dimethyl ester, 1,1-cyclopentanedicarboxylic acid diethyl ester, 1,1-cyclopentanedicarboxylic acid di-n-propyl ester, 1,1-cyclopentanedicarboxylic acid diisopropyl ester, 1,1-cyclopentanedicarboxylic acid di-n-butyl ester, 1,1-cyclopentanedicarboxylic acid diisobutyl ester, 1,1-cyclopentanedicarboxylic acid di-n-pentyl ester, 1,1-cyclopentanedicarboxylic acid di-n-hexyl ester, 1,1-cyclopentanedicarboxylic acid dicyclohexyl ester, 1,1-cyclohexanedicarboxylic acid dimethyl ester, 1,1-cyclohexanedicarboxylic acid diethyl ester, 1,1-cyclohexanedicarboxylic acid di-n-propyl ester, 1,1-cyclohexanedicarboxylic acid diisopropyl ester, 1,1-cyclohexanedicarboxylic acid di-n-butyl ester, 1,1-cyclohexanedicarboxylic acid diisobutyl ester, 1,1-cyclohexanedicarboxylic acid di-n-pentyl ester, 1,1-cyclohexanedicarboxylic acid di-n-hexyl ester, and 1,1-cyclohexanedicarboxylic acid dicyclohexyl ester.

As the compound (8) in which Z is a halogen atom, those produced from commercially available 1,1-cycloalkanedicarboxylic acids by processes described in Australian Journal of Chemistry, vol. 36, pp. 1133-1140 (1983), Organic and Biomolecular Chemistry, vol. 5, pp. 3932-3937 (2007), and the like can be used.

As the compound (8) in which Z is an alkoxy group having 1 to 6 carbon atoms, commercially available compounds and those produced from commercially available malonic acid diesters by methods described in Tetrahedron Letters, vol. 46, pp. 635-638 (2005), and the like can be used.

The reaction of a compound (7) with a compound (8) is preferably conducted by mixing a compound (7) and a compound (8) in the presence of a solvent. When a compound (8) in which Z is a halogen atom is used, a compound (8) is preferably added to a compound (7).

The use amount of a compound (8) is preferably 0.3 to 2 mol, more preferably 0.5 to 1 mol per 1 mol of a compound (7).

Examples of the solvent include solvents inactive against the reaction, and include aromatic hydrocarbon solvents such as toluene and xylene; aliphatic hydrocarbon solvents such as heptane and octane; halogenated hydrocarbon solvents such as chlorobenzene, dichloromethane, dichloroethane and chloroform, used singly or in combination.

The use amount of the solvent is preferably 2 to 200 parts by weight per 1 part by weight of a compound (7).

When a compound (8) in which Z is a halogen atom is used, the reaction temperature is preferably -30 to 100°C, more preferably -10 to 50°C. The reaction is preferably carried out in the presence of a base such as triethylamine, for capturing a generated hydrogen halide. The reaction time is preferably 0.1 to 24 hours, more preferably 2 to 16 hours.

When a compound (8) in which Z is an alkoxy group having 1 to 6 carbon atoms is used, the reaction temperature is preferably 50 to 250°C, more preferably 60 to 180°C. The reaction is preferably carried out in the presence of a catalyst such as a lithium compound. Examples of the lithium compound include lithium alkoxides such as lithium methoxide and lithium ethoxide; lithium halides such as lithium chloride; lithium hydroxide; etc.

The use amount of the catalyst is preferably 0.0005 to 0.5 mol per 1 mol of a compound (8).

The reaction time is preferably 0.5 to 24 hours, more preferably 2 to 16 hours.

The compound (9) is obtained by reacting a compound (7) with a compound (8). The compound (9) can be isolated by mixing the reaction mixture and water and performing extraction, and concentrating the resulting organic layer. The compound (9) can be isolated also by subjecting the reaction mixture itself to column chromatography. The isolated compound (9) can be usually used in the subsequent step without modification. The isolated compound (9) can be purified by subjecting to recrystallization, column chromatography and the like before being used in the reaction of the subsequent step.

Examples of the compound (9) include
N,N'-bis[(1R)-1-(methoxycarbonyl)-2-hydroxyethyl]cyclopropa ne-1,1-dicarboxamide,
N,N'-bis[(1R)-1-(ethoxycarbonyl)-2-hydroxyethyl]cyclopropan e-1,1-dicarboxamide,
N,N'-bis[(1R)-1-(n-propoxycarbonyl)-2-hydroxyethyl]cyclopro pane-1,1-dicarboxamide,
N,N'-bis[(1R)-1-(isopropoxycarbonyl)-2-hydroxyethyl]cyclopr opane-1,1-dicarboxamide,
N,N'-bis[(1R)-1-(n-butoxycarbonyl)-2-hydroxyethyl]cycloprop ane-1,1-dicarboxamide,
N,N'-bis[(1R)-1-(isobutoxycarbonyl)-2-hydroxyethyl]cyclopro pane-1,1-dicarboxamide,
N,N'-bis[(1R)-1-(tert-butoxycarbonyl)-2-hydroxyethyl]cyclop ropane-1,1-dicarboxamide,
N,N'-bis[(1R)-1-(n-pentyloxycarbonyl)-2-hydroxyethyl]cyclop ropane-1,1-dicarboxamide,
N,N'-bis[(1R)-1-(n-hexyloxycarbonyl)-2-hydroxyethyl]cyclopr opane-1,1-dicarboxamide,
N,N'-bis[(1R)-1-(cyclohexyloxycarbonyl)-2-hydroxyethyl]cycl opropane-1,1-dicarboxamide,
N,N'-bis[(1R)-1-(thexyloxycarbonyl)-2-hydroxyethyl]cyclopro pane-1,1-dicarboxamide,
N,N'-bis[(1R)-1-(phenoxycarbonyl)-2-hydroxyethyl]cyclopropa ne-1,1-dicarboxamide,
N,N'-bis[(1R)-1-(benzyloxycarbonyl)-2-hydroxyethyl]cyclopro pane-1,1-dicarboxamide,
N,N'-bis[(1R)-1-(methoxycarbonyl)-2-hydroxyethyl]cyclobutan e-1,1-dicarboxamide,
N,N'-bis[(1R)-1-(ethoxycarbonyl)-2-hydroxyethyl]cyclobutane -1,1-dicarboxamide,
N,N'-bis[(1R)-1-(n-propoxycarbonyl)-2-hydroxyethyl]cyclobut ane-1,1-dicarboxamide,
N,N'-bis[(1R)-1-(isopropoxycarbonyl)-2-hydroxyethyl]cyclobu tane-1,1-dicarboxamide,
N,1,1'-bis[(1R)-1-(n-butoxycarbonyl)-2-hydroxyethyl]cyclobuta ne-1,1-dicarboxamide,
N,N'-bis[(1R)-1-(isobutoxycarbonyl)-2-hydroxyethyl]cyclobut ane-1,1-dicarboxamide,
N,N'-bis[(1R)-1-(tert-butoxycarbonyl)-2-hydroxyethyl]cyclob utane-1,1-dicarboxamide,
N,N'-bis[(1R)-1-(n-pentyloxycarbonyl)-2-hydroxyethyl]cyclob utane-1,1-dicarboxamide,
N,N'-bis[(1R)-1-(n-hexyloxycarbonyl)-2-hydroxyethyl]cyclobu tane-1,1-dicarboxamide,
N,N'-bis[(1R)-1-(cyclohexyloxycarbonyl)-2-hydroxyethyl]cycl obutane-1,1-dicarboxamide,
N,N'-bis[(1R)-1-(thexyloxycarbonyl)-2-hydroxyethyl]cyclobut ane-1,1-dicarboxamide,
N,N'-bis[(1R)-1-(phenoxycarbonyl)-2-hydroxyethyl]cyclobutan e-1,1-dicarboxamide,
N,N'-bis[(1R)-1-(benzyloxycarbonyl)-2-hydroxyethyl]cyclobut ane-1,1-dicarboxamide,
N,N'-bis[(1R)-1-(methoxycarbonyl)-2-hydroxyethyl]cyclopenta ne-1,1-dicarboxamide,
N,N'-bis[(1R)-1-(ethoxycarbonyl)-2-hydroxyethyl]cyclopentan e-1,1-dicarboxamide,
N,N'-bis[(1R)-1-(n-propoxycarbonyl)-2-hydroxyethyl]cyclopen tane-1,1-dicarboxamide,
N,N'-bis[(1R)-1-(isopropoxycarbonyl)-2-hydroxyethyl]cyclope ntane-1,1-dicarboxamide,
N,N'-bis[(1R)-1-(n-butoxycarbonyl)-2-hydroxyethyl]cyclopent ane-1,1-dicarboxamide,
N,N'-bis[(1R)-1-(isobutoxycarbonyl)-2-hydroxyethyl]cyclopen tane-1,1-dicarboxamide,
N,N'-bis[(1R)-1-(tert-butoxycarbonyl)-2-hydroxyethyl]cyclop entane-1,1-dicarboxamide,
N,N'-bis[(1R)-1-(n-pentyloxycarbonyl)-2-hydroxyethyl]cyclop entane-1,1-dicarboxamide,
N,N'-bis[(1R)-1-(n-hexyloxycarbonyl)-2-hydroxyethyl]cyclope ntane-1,1-dicarboxamide,
N,N'-bis[(1R)-1-]cyclohexyloxycarbonyl)-2-hydroxyethyl]cycl opentane-1,1-dicarboxamide,
N,N'-bis[(1R)-1-(thexyloxycarbonyl)-2-hydroxyethyl]cyclopen tane-1,1-dicarboxamide,
N,N'-bis[(1R)-1-(phenoxycarbonyl)-2-hydroxyethyl]cyclopenta ne-1,1-dicarboxamide,
N,N'-bis[(1R)-1-(benzyloxycarbonyl)-2-hydroxyethyl]cyclopen tane-1,1-dicarboxamide,
N,N'-bis[(1R)-1-(methoxycarbonyl)-2-hydroxyethyl]cyclohexan e-1,1-dicarboxamide,
N,N'-bis[(1R)-1-(ethoxycarbonyl)-2-hydroxyethyl]cyclohexane -1,1-dicarboxamide,
N,N'-bis[(1R)-1-(n-propoxycarbonyl)-2-hydroxyethyl]cyclohex ane-1,1-dicarboxamide,
N,N'-bis[(1R)-1-(isopropoxycarbonyl)-2-hydroxyethyl]cyclohe xane-1,1-dicarboxamide,
N,N'-bis[(1R)-1-(n-butoxycarbonyl)-2-hydroxyethyl]cyclohexa ne-1,1-dicarboxamide,
N,N'-bis[(1R)-1-(isobutoxycarbonyl)-2-hydroxyethyl]cyclohex ane-1,1-dicarboxamide,
N,N'-bis[(1R)-1-(tert-butoxycarbonyl)-2-hydroxyethyl]cycloh exane-1,1-dicarboxamide,
N,N'-bis[(1R)-1-(n-pentyloxycarbonyl)-2-hydroxyethyl]cycloh exane-1,1-dicarboxamide,
N,N'-bis[(1R)-1-(n-hexyloxycarbonyl)-2-hydroxyethyl]cyclohe xane-1,1-dicarboxamide,
N,N'-bis[(1R)-1-(cyclohexyloxycarbonyl)-2-hydroxyethyl]cycl ohexane-1,1-dicarboxamide,
N,N'-bis[(1R)-1-(thexyloxycarbonyl)-2-hydroxyethyl]cyclohex ane-1,1-dicarboxamide,
N,N'-bis[(1R)-1-(phenoxycarbonyl)-2-hydroxyethyl]cyclohexan e-1,1-dicarboxamide and
N,N'-bis[(1R)-1-(benzyloxycarbonyl)-2-hydroxyethyl]cyclohex ane-1,1-dicarboxamide;
compounds obtained by replacing the steric configuration (1R) at 1-position of a diamide of each of the above-described compounds with (1S), that is, N,N'-bis[(1S)-1-(methoxycarbonyl)-2-hydroxyethyl]cyclopropa ne-1,1-dicarboxamide and the like;
compounds in which the steric configuration of one of two amide skeletons of each of the above-described compounds is (1R) and the steric configuration of the other is (1S), that is, N-[(1R)-1-(methoxycarbonyl)-2-hydroxyethyl]-N'-[(1S)-1-(met hoxycarbonyl)-2-hydroxyethyl]cyclopropane-1,1-dicarboxamide and the like.

### <Second step>

The second step is a step of reacting the compound (9) with a (methoxycarbonylsulfamoyl)triethylammonium hydroxide intramolecular salt to give an optically active bisoxazoline ester compound represented by formula (11): 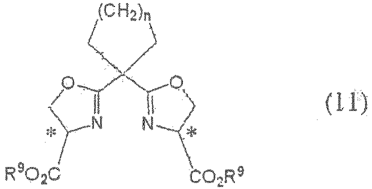 wherein R⁹, n and * are as defined above,
(hereinafter, referred to as compound (11) in some cases.).

The (methoxycarbonylsulfamoyl)triethylammonium hydroxide intramolecular salt is a compound represented by formula (10) : 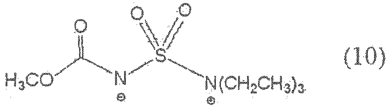 (hereinafter, referred to as compound (10) in some cases), and commercially available as a Burgess reagent.

The reaction of a compound (9) with a compound (10) is preferably conducted by mixing a compound (9) and a compound (10) in the presence of a solvent.

The use amount of the compound (10) is preferably 1 to 10 mol, more preferably 2 to 5 mol per 1 mol of the compound (9).

Examples of the solvent include solvents inactive against the reaction, and include ether solvents such as tetrahydrofuran and dioxane; aromatic hydrocarbon solvents such as toluene and xylene; aliphatic hydrocarbon solvents such as heptane and octane; halogenated hydrocarbon solvents such as chlorobenzene, dichloromethane, dichloroethane and chloroform; used singly or in combination.

The use amount of the solvent is preferably 2 to 200 parts by weight per 1 part by weight of the compound (9).

The reaction temperature is preferably -30 to 120°C, more preferably -20 to 80°C. The reaction time is preferably 0.1 to 24 hours, more preferably 1 to 10 hours.

The compound (11) is obtained by reacting the compound (9) and the compound (10). The resultant compound (11) can be isolated by mixing the reaction mixture and water and performing extraction, and concentrating the resultant organic layer. When the solvent is water-soluble, it may be permissible that the reaction mixture is concentrated, then, a water-insoluble solvent among the above-described solvents or an ester solvent such as ethyl acetate or the like is added and extraction thereof is performed. The compound (11) can also be isolated by subj ecting the reaction mixture itself to column chromatography. The isolated compound (11) can be used in the reaction of the subsequent reaction without modification. The isolated compound (11) can be purified by subjecting to recrystallization, column chromatography and the like before being used in the reaction of the subsequent step.

Examples of the compound (11) include
1,1-bis[2-[(4R)-4-(methoxycarbonyl)oxazoline]]cyclopropane,
1,1-bis[2-[(4R)-4-(ethoxycarbonyl)oxazoline]]cyclopropane,
1,1-bis[2-[(4R)-4-(n-propoxycarbonyl)oxazoline]]cyclopropan e,
1,1-bis[2-[(4R)-4-(isopropoxycarbonyl)oxazoline]]cyclopropa ne,
1,1-bis[2-[(4R)-4-(n-butoxycarbonyl)oxazoline]]cyclopropane ,
1,1-bis[2-[(4R)-4-(isobutoxycarbonyl)oxazoline]]cyclopropan e,
1,1-bis[2-[(4R)-4-(tert-butoxycarbonyl)oxazoline]]cycloprop ane,
1,1-bis[2-[(4R)-4-(n-pentyloxycarbonyl)oxazoline]]cycloprop ane,
1,1-bis[2-[(4R)-4-(n-hexyloxycarbonyl)oxazoline]]cyclopropa ne,
1,1-bis[2-[(4R)-4-(cyclohexyloxycarbonyl)oxazoline]]cyclopr opane,
1,1-bis[2-[(4R)-4-(thexyloxycarbonyl)oxazoline]]cyclopropan e,
1,1-bis[2-[(4R)-4-(phenoxycarbonyl)oxazoline]]cyclopropane,
1,1-bis[2-[(4R)-4-(benzyloxycarbonyl)oxazoline]]cyclopropan e,
1,1-bis[2-[(4R)-4-(methoxycarbonyl)oxazoline]]cyclobutane,
1,1-bis[2-[(4R)-4-(ethoxycarbonyl)oxazoline]]cyclobutane,
1,1-bis[2-[(4R)-4-(n-propoxycarbonyl)oxazoline]]cyclobutane ,
1,1-bis[2-[(4R)-4-(isopropoxycarbonyl)oxazoline]]cyclobutan e,
1,1-bis[2-[(4R)-4-(n-butoxycarbonyl)oxazoline]]cyclobutane,
1,1-bis[2-[(4R)-4-(isobutoxycarbonyl)oxazoline]]cyclobutane ,
1,1-bis[2-[(4R)-4-(tert-butoxycarbonyl)oxazoline]]cyclobuta ne,
1,1-bis[2-[(4R)-4-(n-pentyloxycarbonyl)oxazoline]]cyclobuta ne,
1,1-bis[2-[(4R)-4-(n-hexyloxycarbonyl)oxazoline]]cyclobutan e,
1,1-bis[2-[(4R)-4-(cyclohexyloxycarbonyl)oxazoline]]cyclobu tane,
1,1-bis[2-[(4R)-4-(thexyloxycarbonyl)oxazolinel]cyclobutane ,
1,1-bis[2-[(4R)-4-(phenoxycarbonyl)oxazoline]]cyclobutane,
1,1-bis[2-[(4R)-4-(benzyloxycarbonyl)oxazoline]]cyclobutane ,
1,1-bis[2-[(4R)-4-(methoxycarbonyl)oxazoline]]cyclopentane,
1,1-bis[2-[(4R)-4-(ethoxycarbonyl)oxazoline]]cyclopentane,
1,1-bis[2-[(4R)-4-(n-propoxycarbonyl)oxazoline]]cyclopentan e,
1,1-bis[2-[(4R)-4-(isopropoxycarbonyl)oxazoline]]cyclopenta ne,
1,1-bis[2-[(4R)-4-(n-butoxycarbonyl)oxazoline]]cyclopentane,
1,1-bis[2-[(4R)-4-(isobutoxycarbonyl)oxazoline]]cyclopentan e,
1,1-bis[2-[(4R)-4-(tert-butoxycarbonyl)oxazoline]]cyclopent ane,
1,1-bis[2-[(4R)-4-(n-pentyloxycarbonyl)oxazoline]]cyclopent ane,
1,1-bis[2-[(4R)-4-(n-hexyloxycarbonyl)oxazoline]]cyclopenta ne,
1,1-bis[2-[(4R)-4-(cyclohexyloxycarbonyl)oxazoline]]cyclope ntane,
1,1-bis[2-[(4R)-4-(thexyloxycarbonyl)oxazoline]]cyclopentan e,
1,1-bis[2-[(4R)-4-(phenoxycarbonyl)oxazoline]]cyclopentane,
1,1-bis[2-[(4R)-4-(benzyloxycarbonyl)oxazoline]]cyclopentan e,
1,1-bis[2-[(4R)-4-(methoxycarbonyl)oxazoline]]cyclohexane,
1,1-bis[2-[(4R)-4-(ethoxycarbonyl)oxazoline]]cyclohexane,
1,1-bis[2-[(4R)-4-(n-propoxycarbonyl)oxazoline]]cyclohexane,
1,1-bis[2-[(4R)-4-(isopropoxycarbonyl)oxazoline]]cyclohexan e,
1,1-bis[2-[(4R)-4-(n-butoxycarbonyl)oxazoline]]cyclohexane,
1,1-bis[2-[(4R)-4-(isobutoxycarbonyl)oxazoline]]cyclohexane ,
1,1-bis[2-[(4R)-4-(tert-butoxycarbonyl)oxazoline]]cyclohexa ne,
1,1-bis[2-[(4R)-4-(n-pentyloxycarbonyl)oxazoline]]cyclohexa ne,
1,1-bis[2-[(4R)-4-(n-hexyloxycarbonyl)oxazoline]]cyclohexan e,
1,1-bis[2-[(4R)-4-(cyclohexyloxycarbonyl)oxazoline]]cyclohe xane,
1,1-bis[2-[(4R)-4-(thexyloxycarbonyl)oxazoline]]cyclohexane and
1,1-bis[2-[(4R)-4-(phenoxycarbonyl)oxazoline]]cyclopentane, 1,1-bis[2-[(4R)-4-(benzyloxycarbonyl)oxazoline]]cyclohexane;
compounds obtained by replacing the steric configuration (4R) at 4-position of an oxazoline ring of each of the above-described compounds with (4S), that is, 1,1-bis[2-[(4S)-4-(methoxycarbonyl)oxazoline]]cyclopropane, and the like; and
compounds in which the steric configuration of one of two oxazoline skeletons of each of the above-described compounds is (4R) and the steric configuration of the other is (4S), that is, 1-[2-[(4R)-4-(methoxycarbonyl)oxazoline]]-1-[2-[(4S)-4-(met hoxycarbonyl)oxazoline]]cyclopropane and the like.

### <Third step>

The third step is a step of reacting the compound (11) with a Grignard reagent represented by formula (12):

CH₃MgQ (12)

wherein Q represents a halogen atom,
(hereinafter, referred to as compound (12) in some cases.) to give an optically active bisoxazoline alcohol compound represented by formula (13): 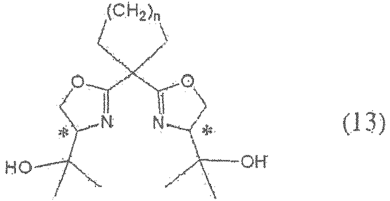 wherein n and * are as defined above,
(hereinafter, referred to as compound (13) in some cases.).

Examples of the halogen atom represented by Q include a chlorine atom and a bromine atom. As the compound (12), commercially available compounds can be used.

Examples of the compound (12) include methylmagnesium bromide.

The reaction of the compound (11) with the compound (12) is preferably conducted by mixing the compound (11) and the compound (12) in the presence of a solvent.

The use amount of the compound (12) is preferably 2 to 15 mol, more preferably 4 to 8 mol per 1 mol of the compound (11).

As the solvent, ether solvents such as diethyl ether, tetrahydrofuran and dioxane are preferably used, and a mixed solvent with an aromatic hydrocarbon solvent inactive against the reaction such as toluene and xylene may also be used.

The use amount of the solvent is preferably 2 to 200 parts by weight per 1 part by weight of the compound (11).

The reaction temperature is preferably -80 to 120°C, more preferably -70 to 20°C. The reaction time is preferably 0.5 to 24 hours, more preferably 1 to 10 hours.

The compound (13) is obtained by reacting the compound (11) with the compound (12). The resultant compound (13) can be isolated by mixing the reaction mixture and an aqueous solution of a weak acid such as ammonium chloride and performing extraction, and concentrating the resulting organic layer. The isolated compound (13) can be used in the reaction of the subsequent step without modification. The isolated compound (13) can be purified by subjecting to recrystallization, column chromatography and the like before being used in the reaction of the subsequent step.

Examples of the compound (13) include
1,1-bis[2-[(4R)-4-(2-hydroxy-2-propyl)oxazoline]]cyclopropa ne,
1,1-bis[2-[(4R)-4-(2-hydroxy-2-propyl)oxazoline]]cyclobutan e,
1,1-bis[2-[(4R)-4-(2-hydroxy-2-propyl)oxazoline]]cyclopenta ne and
1,1-bis[2-[(4R)-4-(2-hydroxy-2-propyl)oxazoline]]cyclohexan e;
compounds obtained by replacing the steric configuration (4R) at 4-position of an oxazoline ring of each of the above-described compounds with (4S), that is, 1,1-bis[2-[(4S)-4-(2-hydroxy2-propyl)oxazoline]]cyclopropan e, and the like; and
compounds in which the steric configuration of one of two oxazoline skeletons of each of the above-described compounds is (4R) and the steric configuration of the other is (4S), that is, 1-[2-[(4R)-4-(2-hydroxy2-propyl)oxazoline]]-1-[2-[(4S)-4-(2 -hydroxy2-propyl)oxazoline]]cyclopropane and the like.

### <Fourth step>

The fourth step is a step of reacting the compound (13) with a silicon compound represented by formula (14): 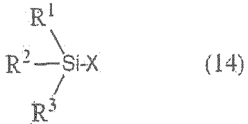 wherein R¹, R² and R³ are as defined above and X represents a halogen atom,
(hereinafter, referred to as compound (14) in some cases.) to give a compound (1).

Examples of the halogen atom represented by X include a chlorine atom and a bromine atom.

As the compound (14), commercially available compounds and those obtained by purifying commercially available compounds by distillation and the like are used.

Examples of the compound (14) include triisopropylsilyl chloride, thexyldimethylsilyl chloride, triethylsilyl chloride, triphenylsilyl chloride, tert-butyldiphenylsilyl chloride and methyldiphenylsilyl chloride.

The reaction of the compound (13) with the compound (14) is preferably conducted by mixing the compound (13) and the compound (14) in the presence of a base and a solvent.

The reaction of the compound (13) with the compound (14) is preferably conducted by mixing the compound (13) and a base, then, mixing the compound (14).

The use amount of the compound (14) is preferably 1 to 10 mol, more preferably 2 to 6 mol per 1 mol of the compound (13).

Examples of the solvent include solvents inactive against the reaction, and include ether solvents such as tetrahydrofuran and dioxane; aromatic hydrocarbon solvents such as toluene and xylene; aliphatic hydrocarbon solvents such as heptane and octane; used singly or in combination.

The use amount of the solvent is preferably 2 to 200 parts by weight per 1 part by weight of the compound (13).

Examples ot the base include organometal compounds such as n-butyllithium and lithium diisopropylamide; metal hydrides such as lithium hydride, sodium hydride, potassium hydride and calcium hydride; metal alkoxides such as lithium methoxide, lithium ethoxide, sodium methoxide, sodium ethoxide, sodium t-butoxide, potassium methoxide and potassium t-butoxide; metal hydroxides such as lithium hydroxide, sodium hydroxide, potassium hydroxide and calcium hydroxide; metal carbonates such as lithium carbonate, sodium carbonate, potassium carbonate and calcium carbonate; etc.

The use amount of the base is preferably 1 to 10 mol, more preferably 2 to 6 mol per 1 mol of the compound (13).

The reaction temperature is preferably -80 to 120°C, more preferably -20 to 80°C. The reaction time is preferably 0.5 to 24 hours, more preferably 1 to 16 hours.

The compound (1) is obtained by reacting the compound (13) with the compound (14). The resultant compound (1) can be isolated by mixing the reaction mixture and an aqueous solution of a weak acid such as ammonium chloride and performing extraction, and concentrating the resulting organic layer. The resultant compound (1) can be isolated by treating the reaction mixture with alcohols such as methanol and ethanol, then, treating by column chromatography. The resultant compound (1) can be isolated by treating the reaction mixture itself by column chromatography. The isolated compound (1) can be used as itself for preparation of an asymmetric catalyst, or, if necessary, can be purified by subjecting to recrystallization, column chromatography and the like before being used for preparation of an asymmetric catalyst.

### <<Asymmetric catalyst»

The asymmetric catalyst of the present invention is obtained by mixing the following (A) and (B).
(A) a monovalent or divalent copper compound (hereinafter, referred to as component (A) in some cases.)
(B) a compound (1) (hereinafter, referred to as component

### (B) in some cases.)

Examples of the component (A) include monovalent or divalent copper compounds such as copper(I) trifluoromethanesulfonate, copper(I) acetate, copper(I) bromide, copper(I) chloride, copper(I) iodide, copper(I) hydroxide, copper(II) trifluoromethanesulfonate, copper(II) acetate, copper(II) bromide, copper(II) chloride, copper(II) iodide and copper(II) hydroxide, and copper(I) trifluoromethanesulfonate, copper(I) chloride, copper(I) bromide, copper(I) iodide, copper(II) chloride, copper(II) bromide or copper(II) iodide is preferred. The copper compounds may each be used singly or two or more of them may be used in admixture.

As the component (A), commercially available compounds can be used. As the component (A), monovalent copper compounds and monovalent copper compounds generated by allowing a reducing agent such as phenylhydrazine to act on divalent copper compounds are preferably used, from the standpoint of reaction efficiency.

The use amount of the component (B) is preferably 0.8 to 5 mol, more preferably 0.9 to 2 mol per 1 mol of the component (A).

Mixing of the component (A) and the component (B) is preferably carried out in the presence of a solvent. Examples of the solvent include halogenated hydrocarbon solvents such as dichloromethane, dichloroethane, chloroform and carbon tetrachloride; aromatic hydrocarbon solvents such as benzene, toluene and xylene; ester solvents such as methyl acetate and ethyl acetate; etc.

When the prochiral olefin represented by formula (4) (hereinafter, abbreviated as olefin (4)) as a raw material of an asymmetric cyclopropanation reaction described later is liquid, the olefin (4) maybe used as a solvent. The use amount of the solvent is preferably 10 to 10000 parts by weight per 1 part by weight of the component (A).

Mixing of the component (A) and the component (B) is preferably carried out under an atmosphere of an inert gas such as an argon gas and a nitrogen gas, and the mixing temperature is preferably -20 to 100°C.

The asymmetric catalyst of the present invention is preferably obtained by further mixing the following (C) or (D), from the standpoint of activity of the asymmetric catalyst.

### (C) a boron compound represented by formula (2):

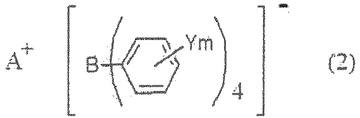

wherein A⁺ represents a lithium ion, a sodium ion, a potassium ion, a silver (I) ion or a trityl cation, Y represents a fluorine atom or a fluorinated alkyl group having 1 to 8 carbon atoms, m represents an integer of 1 to 5, and when m is 2 or more, the plurality of Y are the same or different,
(hereinafter, referred to as component (C) in some cases.)

### (D) a fluorine compound represented by formula (3):

D⁺ (MF₆)⁻ (3)

wherein D⁺ represents a lithium ion, a sodium ion, a potassium ion, a silver(I) ion or a trityl cation and M represents a phosphorus atom, an arsenic atom or an antimony atom.
(hereinafter, referred to as component (D) in some cases.)

In the fomula of the boron compound represented by formula (2) (hereinafter, referred to as boron compound (2) in some cases.), A⁺ represents a lithium ion, a sodium ion, a potassium ion, a silver(I) ion or a trityl cation, preferably a trityl cation.

Y represents a fluorine atom or a fluorinated alkyl group having 1 to 8 carbon atoms. m is an integer of 1 to 5 representing the number of the substituent Y. The substituent Y may be substituted on any position on a phenyl group. When m is 2 or more, the plurality of Y are the same or different.

Examples of the fluorinated alkyl group having 1 to 8 carbon atoms include alkyl groups obtained by substituting at least one hydrogen atom on an alkyl group having 1 to 8 carbon atoms by a fluorine atom such as a fluoromethyl group, a difluoromethyl group, a trifluoromethyl group, a perfluoroethyl group, a perfluoro-n-propyl group, a perfluoro-n-butyl group, a perfluoro-n-pentyl group, a perfluoro-n-hexyl group, a perfluoro-n-heptyl group and a perfluoro-n-octyl group.

Examples of the boron compound (2) include
lithium tetrakis(4-fluorophenyl) borate,
sodium tetrakis(4-fluorophenyl) borate,
potassium tetrakis(4-fluorophenyl) borate,
silver tetrakis(4-fluorophenyl) borate,
trityl tetrakis(4-fluorophenyl) borate,
lithium tetrakis(3,5-difluorophenyl) borate,
sodium tetrakis(3,5-difluorophenyl) borate,
potassium tetrakis(3,5-difluorophenyl) borate,
silver tetrakis(3,5-difluorophenyl) borate,
trityl tetrakis(3,5-difluorophenyl) borate,
lithium tetrakis(3,4,5-trifluorophenyl) borate,
sodium tetrakis(3,4,5-trifluorophenyl) borate,
potassium tetrakis(3,4,5-trifluorophenyl),
silver tetrakis(3,4,5-trifluorophenyl) borate,
trityl tetrakis(3,4,5-trifluorophenyl) borate,
lithium tetrakis(pentafluorophenyl) borate,
sodium tetrakis(pentafluorophenyl) borate,
potassium tetrakis(pentafluorophenyl) borate,
silver tetrakis(pentafluorophenyl) borate,
trityl tetrakis(pentafluorophenyl) borate,
lithium tetrakis[3,5-bis(trifluoromethyl)phenyl] borate,
sodium tetrakis[3,5-bis(trifluoromethyl)phenyl] borate,
potassium tetrakis[3,5-bis(trifluoromethyl)phenyl] borate,
silver tetrakis[3,5-bis(trifluoromethyl)phenyl] borate,
trityl tetrakis[3,5-bis(trifluoromethyl)phenyl] borate,
lithium tetrakis[2,4,6-tris(trifluoromethyl)phenyl] borate,
sodium tetrakis[2,4,6-tris(trifluoromethyl)phenyl] borate,
potassium tetrakis[2,4,6-tris(trifluoromethyl)phenyl] borate,
silver tetrakis[2,4,6-tris(trifluoromethyl)phenyl] borate,
trityl tetrakis[2,4,6-tris(trifluoromethyl)phenyl] borate,
lithium tetrakis[pentakis(trifluoromethyl)phenyl] borate,
sodium tetrakis[pentakis(trifluoromethyl)phenyl] borate,
potassium tetrakis[pentakis(trifluoromethyl)phenyl] borate,
silver tetrakis[pentakis(trifluoromethyl)phenyl] borate and
trityl tetrakis[pentakis(trifluoromethyl)phenyl] borate.

As the boron compound (2), commercially available compounds may be used, and those produced by known methods described in JP-A No. 9-295984 and the like may also be used.

In the fluorine compound represented by formula (3) (hereinafter, referred to as fluorine compound (3) in some cases.) as the component (D), A⁺ represents a lithium ion, a sodium ion, a potassium ion, a silver(I) ion or a trityl cation. M represents a phosphorus atom, an arsenic atom or an antimony atom, preferably a phosphorus atom or an antimony atom.

Examples of the fluorine compound (3) include lithium hexafluorophosphate, sodium hexafluorophosphate, potassium hexafluorophosphate, silver hexafluorophosphate, trityl hexafluorophosphate, lithium hexafluoroarsenate, sodium hexafluoroarsenate, potassium hexafluoroarsenate, silver hexafluoroarsenate, trityl hexafluoroarsenate, lithium hexafluoroantimonate, sodium hexafluoroantimonate, potassium hexafluoroantimonate, silver hexafluoroantimonate and trityl hexafluoroantimonate.

The use amount of the component (C) or the component (D) is preferably 0.8 to 5 mol, more preferably 0.9 to 2 mol per 1 mol of the component (A).

When the component (C) or the component (D) is mixed, it is preferable that the component (A) and the component (B) are mixed in the above-described solvent, and further, the component (C) or the component (D) is added.

The asymmetric catalyst of the present invention may be isolated from the mixture obtained by the above-described mixing and used in the reaction of the olefin (4) with a diazoacetate ester represented by the (5) (hereinafter, referred to as diazoacetate ester (5) in some cases.), or the mixture itself may be used in the reaction of the olefin (4) with the diazoacetate ester (5).

### <Asymmetric cyclopropanation reaction>

The step of reacting a prochiral olefin represented by formula (4): 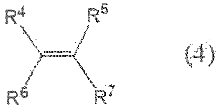 wherein R⁴, R⁵, R⁶ and R⁷ each independently represent a hydrogen atom, a halogen atom, an alkyl group having 1 to 6 carbon atoms and optionally having a substituent, an alkenyl group having 2 to 6 carbon atoms and optionally having a substituent, an aryl group having 6 to 10 carbon atoms and optionally having a substituent, an aralkyl group having 7 to 16 carbon atoms and optionally having a substituent or an alkoxycarbonyl group having 2 to 7 carbon atoms and optionally having a substituent, and when R⁴ and R⁶ represent the same group, R⁵ and R⁷ represent groups different from each other,
with a diazoacetate ester represented by formula (5):

N₂CHCO₂R⁸ (5)

wherein R⁸ represents an alkyl group having 1 to 6 carbon atoms, in the presence of the asymmetric catalyst of the present invention will be explained.

Examples of the halogen atom represented by R⁴, R⁵, R⁶ and R⁷ include a fluorine atom, a chlorine atom, a bromine atom and an iodine atom.

Examples of the alkyl group having 1 to 6 carbon atoms and optionally having a substituent include alkyl groups having 1 to 6 carbon atoms such as a methyl group, an ethyl group, a n-propyl group, an isopropyl group, a n-butyl group, an isobutyl group, a n-pentyl group, and those obtained by substituting one or two or more hydrogen atoms of these alkyl groups by various substituents, and include alkyl groups substituted with a halogen atom such as a chloromethyl group, a fluoromethyl group, a trifluoromethyl group and a chloroethyl group; alkyl groups substituted with an alkoxy group such as a methoxymethyl group, an ethoxymethyl group, a n-propoxymethyl group, an isopropoxymethyl group, a n-butoxymethyl group and a tert-butoxymethyl group ; alkyl groups substituted with an aralkyloxy group such as a benzyloxymethyl; alkyl groups substituted with an acyloxy group such as an acetoxymethyl group and a benzoyloxymethyl group; alkyl groups substituted with an alkoxycarbonyloxy group such as a methoxycarbonyloxymethyl group, an ethoxycarbonyloxymethyl group and a tert-butoxycarbonyloxymethyl group; alkyl groups substituted with an aryloxycarbonyloxy group such as a phenyloxycarbonyloxymethyl group; etc.

Examples of the alkenyl group having 2 to 6 carbon atoms and optionally having a substituent include alkenyl groups having 2 to 6 carbon atoms such as a vinyl group, a 1-propenyl group, a 2-propenyl group, a 2-methyl-1-propenyl group, a 1-butenyl group, a 2-butenyl group and a 3-butenyl group, and compounds obtained by substituting one or two or more hydrogen atoms on these alkenyl groups by a halogen atom or an alkoxycarbonyl group, that is, a 1-chloro-2-propenyl group, a 2-methoxycarbonyl-1-propenyl group and the like.

Examples of the aryl group having 6 to 10 carbon atoms and optionally having a substituent include a phenyl group, a 1-naphthyl group, a 2-naphthyl group, a 2-methylphenyl group, a 4-methylphenyl group, a 3-methoxymethylphenyl group and a 2,3-dihydro-4-benzofuranyl group.

Examples of the aralkyl group having 7 to 16 carbon atoms and optionally having a substituent include those constituted of the above-described aryl group having 6 to 10 carbon atoms and optionally having a substituent and an alkyl group having 1 to 6 carbon atoms and optionally having a substituent, and include a benzyl group, a 2-methylbenzyl group, a 3-methylbenzyl group, a 4-methylbenzyl group, a 2-methoxybenzyl group, a 3-methoxybenzyl group, a 4-methoxybenzyl group, a 1-naphthylmethyl group and a 2-naphthylmethyl group.

Examples of the alkoxycarbonyl group having 2 to 7 carbon atoms and optionally having a substituent include those constituted of the above-described alkyl group having 1 to 6 carbon atoms and optionally having a substituent, an oxygen atom and a carbonyl group, and include a methoxycarbonyl group, an ethoxycarbonyl group, a n-propyloxycarbonyl group, an isopropyloxycarbonyl group, a n-butyloxycarbonyl group, an isobutyloxycarbonyl group and a n-pentyloxycarbonyl group.

In formula (4), when R⁴ and R⁶ represent the same group, R⁵ and R⁷ represent groups different from each other.

R⁴ is preferably a hydrogen atom or a fluorine atom, R⁵ is preferably a methyl group, R⁶ is preferably an acyloxymethyl group, an alkoxycarbonyloxymethyl group or an aralkyloxymethyl group, an R⁷ is preferably a methyl group. R⁶ is more preferably an acetoxymethyl group or a benzyloxymethyl group.

Examples of the olefin (4) include propene, fluoroethylene, 1-fluoro-1-chloroethylene, 1-butene, isobutene, 1-pentene, 1-hexene, 1-octene, 4-chloro-1-butene, 2-pentene, 2-heptene, 2-methyl-2-butene, 2,5-dimethyl-2,4-hexadiene, 2-chloro-5-methyl-2,4-hexadiene, 2-fluoro-5-methyl-2,4-hexadiene, 1,1,1-trifluoro-5-methyl-2,4-hexadiene, 2-methoxycarbonyl-5-methyl-2,4-hexadiene, 1,1-difluoro-4-methyl-1,3-pentadiene, 1,1-dichloro-4-methyl-1,3-pentadiene, 1,1-dibromo-4-methyl-1,3-pentadiene, 1-chloro-1-fluoro-4-methyl-1,3-pentadiene, 1-fluorn-1-bromo-4-methyl-1,3-pentadiene, 2-methyl-2,4-hexadiene, 1-fluoro-1,1-dichloro-4-methyl-2-pentene, 1,1,1-trichloro-4-methyl-3-pentene, 1,1,1-tribromo-4-methyl-3-pentene, 2,3-dimethyl-2-pentene, 2-methyl-3-phenyl-2-butene, 2-bromo-2,5-dimethyl-4-hexene, 2-chloro-2,5-dimethyl-4-hexene, 1-chloro-2,5-dimethyl-2,4-hexadiene, (3-methyl-2-butenyl)methyl ether, (3-methyl-2-butenyl)tert-butyl ether, (3-methyl-2-butenyl)benzyl ether, 3-methyl-2-butenyl acetate, 3-methyl-2-butenyl benzoate, (3-methyl-2-butenyl)methyl carbonate, (3-methyl-2-butenyl)tert-butyl carbonate, (3-methyl-2-butenyl)phenyl carbonate, styrene and 4-vinyl-2,3-dihydrobenzofuran and the like.

R⁸ in formula (5) represents an alkyl group having 1 to 6 carbon atoms, and examples of the alkyl group include a methyl group, an ethyl group, a n-propyl group, an isopropyl group, a n-butyl group, an isobutyl group and a n-pentyl group.

Examples of the diazoacetate ester (5) include ethyl diazoacetate, n-propyl diazoacetate, isopropyl diazoacetate, n-butyl diazoacetate, isobutyl diazoacetate and tert-butyl diazoacetate.

As the diazoacetate ester (5), those produced by known methods described in Organic Synthesis Collective Volume 3, p. 392 and the like can be used.

The use amount of the asymmetric catalyst is preferably 0.00001 to 0.5 mol, more preferably 0.0001 to 0.05 mol in terms of copper metal per 1 mol of the diazoacetate ester (5).

The use amount of the olefin (4) is preferably 1 mol or more, more preferably 1.2-fold mol or more per 1 mol of the diazoacetate ester (5). Its upper limit is not particularly restricted, and when the olefin (4) is liquid, it may be used in large excess amount also as a solvent.

The reaction of the olefin (4) with the diazoacetate ester (5) is preferably carried out in the presence of a solvent. Examples of the solvent include halogenated hydrocarbon solvents such as dichloromethane, dichloromethane, chloroform and carbon tetrachloride; aliphatic hydrocarbon solvents such as hexane, heptane and cyclohexane; aromatic hydrocarbon solvents such as benzene, toluene and xylen; ester solvents such as ethyl acetate, used singly or in combination.

The use amount of the solvent is preferably 1 to 30 parts by weight, more preferably 2 to 20 parts by weight per 1 part by weight of the diazoacetate ester (5), from the standpoint of volumetric efficiency, the property and condition of the reaction mixture and the like.

The reaction of the olefin (4) with the diazoacetate ester (5) is preferably carried out under an atmosphere of an inert gas such as an argon gas and a nitrogen gas. Since water exerts a reverse influence on the reaction, it is preferable to suppress the water content present in the reaction system low by having out the reaction in the co-existence of a dehydrating agent in the reaction system or by using the olefin (4) and a solvent previously dehydrated and the like.

The reaction temperature is preferably -50 to 150°C, more preferably -20 to 80°C.

The reaction of the olefin (4) with the diazoacetate ester (5) is usually carried out by mixing the asymmetric catalyst, the olefin (4) and the diazoacetate ester (5), if necessary in the presence of a solvent, and it is preferable that the reaction is carried out by mixing the asymmetric catalyst and the olefin (4) in a solvent, and adding the diazoacetate ester (5).

By such a reaction, an optically active cyclopropane compound represented by formula (6): 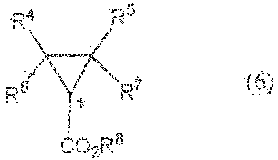 wherein each of R⁴, R⁵, R⁶, R⁷ and R⁸ are as defined above and
* represents an asymmetric center,
(hereinafter, referred to as optically active cyclopropane compound (6) in some cases.)
is obtained.

The optically active cyclopropane compound (6) can be isolated by concentrating the reaction mixture after completion of the reaction. The resultant optically active cyclopropane compound (6) may be further purified by usual purification means such as distillation and column chromatography. The process for producing an optically active cyclopropane compound (6) of the present invention may include these isolation steps and purification steps.

Examples of the optically active cyclopropane compound (6) include
optically active methyl 2-fluorocyclopropanecarboxylate,
optically active methyl 2-fluoro2-chlorocyclopropanecarboxylate,
optically active methyl 2-methylcyclopropanecarboxylate,
optically active methyl 2,2-dimethylcyclopropanecarboxylate,
optically active methyl 2,2-dimethyl-3-(2-methyl-1-propenyl)cyclopropanecarboxylate,
optically active methyl 2,2-dimethyl-3-(2,2-dichloro-1-ethenyl)cyclopropanecarboxyl ate,
optically active methyl 2,2-dimethyl-3-(2,2,2-trichloroethyl)cyclopropanecarboxylat e,
optically active methyl 2,2-dimethyl-3-(2,2,2-tribromoethyl)cyclopropanecarboxylate ,
optically active methyl 2,2-dimethyl-3-(2,2-dibromo-1-ethenyl)cyclopropanecarboxyla te,
optically active methyl 2,2-dimethyl-3-(2,2-difluoro-1-ethenyl)cyclopropanecarboxyl ate,
optically active methyl 2,2-dimethyl-3-(2-fluoro-2-chloro-1-ethenyl)cyclopropanecar boxylate,
optically active methyl 2,2-dimethyl-3-(2-fluoro-2-bromo-1-ethenyl)cyclopropanecarb oxylate,
optically active methyl 2,2-dimethyl-3-(2-fluoro-1-propenyl)cyclopropanecarboxylate,
optically active methyl 2,2-dimethyl-3-(2-chloro-1-propenyl)cyclopropanecarboxylate ,
optically active methyl 2,2-dimethyl-3-(2-chloro-2-trifluoromethylethenyl)cycloprop anecarboxylate,
optically active methyl 2,2-dimethyl-3-(2-methoxycarbonyl-1-propenyl)cyclopropaneca rboxylate,
optically active methyl 2,2-dimethyl-3-(2-chloro-2-methylpropyl)cyclopropanecarboxy late,
optically active methyl 2,2-dimethyl-3-(2-bromo-2-methylpropyl)cyclopropanecarboxyl ate,
optically active methyl 2,2-dimethyl-3-(1-propenyl)cyclopropanecarboxylate,
optically active methyl 3,3-dimethyl-2-(methoxymethyl)cyclopropanecarboxylate,
optically active methyl 3,3-dimethyl-2-(tert-butoxymethyl)cyclopropanecarboxylate,
optically active methyl 3,3-dimethyl-2-(benzyloxymethyl)cyclopropanecarboxylate,
optically active methyl 3,3-dimethyl-2-(acetoxymethyl)cyclopropanecarboxylate,
optically active methyl 3,3-dimethyl-2-(benzoyloxymethyl)cyclopropanecarboxylate,
optically active methyl 3,3-dimethyl-2-(methoxycarbonyloxymethyl)cyclopropanecarbox ylate,
optically active methyl 3,3-dimethyl-2-(tert-butoxycarbonyloxymethyl)cyclopropaneca rboxylate,
optically active methyl 3,3-dimethyl-2-(phenoxycarbonyloxymethyl)cyclopropanecarbox ylate,
optically active methyl 2-phenylcyclopropanecarboxylate and
optically active methyl 2-(2,3-dihydro-4-benzofuranyl)cyclopropanecarboxylate; and
compounds obtained by converting the above-described methyl ester into an ethyl ester, a n-propyl ester, an isopropyl ester, an isobutyl ester, a tert-butyl ester and the like.

The optically active cyclopropane compound (6) can also be converted into an optically active cyclopropanecarboxylic acid compound represented by formula (6) in which R⁸ is a hydrogen atom by hydrolyzing it according to a known hydrolysis method.

### [EXAMPLES]

The present invention will be illustrated further in detail by examples below.

In each example of production of an optically active bisoxazoline compound, the gas chromatography area percentage is a value determined by using the following conditions.
Column: DB-1 (0.25 µm, 0.25 mmϕ × 30 m) manufactured by J&W
Carrier gas: He 1.0 mL/min, split ratio 1/50
Injection port: 250°C
Detector: FID, 300°C
Column oven: 50°C (0 min) to 300°C at a temperature rising rate of 10°C/min (kept for 10 to 200 minutes)

In each example of the asymmetric cyclopropanation reaction, the yield was determined by a gas chromatography internal standard method and the trans isomer/cis isomer ratio was determined by the gas chromatography area ratio. The optical purity was determined by the liquid chromatography area ratio.

### Example 1-1: Production example of compound (9) (n is 0 and R⁹ is a methyl group)

A 100 mL round-bottomed four-necked flask equipped with a Dimroth condenser having a nitrogen introduction tube, a thermometer, a magnetic rotor and a dropping funnel was purged with nitrogen, then, into the flask were charged 4.04 g (25.5 mmol) of D-serine methyl ester hydrochloride and 40 mL of dehydrated chloroform, and the mixture was cooled to 0°C. Into the resultant mixture, 5.58 g (55.1 mmol) of triethylamine was dropped at 2 to 4°C over a period of 15 minutes, then, a mixed solution of 2.13 g (12.7 mmol) of 1,1-cyclopropanedicarboxylic acid dichloride and 8 mL of dehydrated chloroform was dropped at 2 to 4°C over a period of 1 hour. The resultant reaction mixture was stirred at 2 to 4°C for 1 hour, then, the resultant mixture was heated up to room temperature, and stirred overnight at room temperature. The resultant reaction mixture was purified by silica gel column chromatography (silica gel 120 g, hexane/ethyl acetate/tetrahydrofuran = 90/10/0 to 0/0/100), to give 3.10 g of N,N'-bis[(1R)-1-(methoxycarbonyl)-2-hydroxyethyl]-1,1-cyclo propanedicarboxamide as a white crystal. Yield: 73%.
¹H-NMR (300 MHz, DMSO-d6, tetramethylsilane (TMS) reference) δ (ppm): 8.56 (2H,d), 5.08 (2H,t), 4.40 to 4.34 (2H, m), 3.77 to 3.61 (4H, m), 3.64 (6H, s), 1.39 (4H, s)
¹³C-NMR (75 MHz, CDCl₃, TMS reference)
δ (ppm): 170.80, 170.49, 60.87, 55.08, 51.97, 27.31, 16.58

### Example 1-2: Production example of compound (11) (n is 0 and R⁹ is a methyl group)

A 200 mL round-bottomed four-necked flask equipped with a Dimroth condenser having a nitrogen introduction tube, a thermometer and a magnetic rotor was purged with nitrogen, then, into the flask were charged 3.06 g (9.21 mmol) of N,N'-bis[(1R)-1-(methoxycarbonyl)-2-hydroxyethyl]-1,1-cyclo propanedicarboxamide and 92 mL of dehydrated tetrahydrofuran, and the mixture was cooled to 0°C. To the resultant mixture was added 4.71 g (19.2 mmol) of a (methoxycarbonylsulfamoyl)triethylammonium hydroxide intramolecular salt at 0°C, then, the mixture was stirred at 0°C for 10 minutes. The resultant mixture was heated until starting reflux thereof, and refluxed for 2.5 hours at the same temperature, then, cooled down to room temperature. The resultant reaction mixture was concentrated, and to the resultant residue were added 153 mL of chloroform and 15 mL of a 5 wt% sodium hydrogen carbonate aqueous solution, and these were mixed, then, the liquid was separated. The resultant organic layer was washed with 15 mL of saturated saline, then, the liquid was separated, and the resultant organic layer was dried over anhydrous sodium sulfate. Sodium sulfate was removed by filtration, then, the mixture was concentrated, and the resultant residue was purified by silica gel column chromatography (silica gel 250 g, hexane/ethyl acetate/tetrahydrofuran = 20/20/1 to 5/5/1), to give 1.14 g of colorless oily 1,1-bis[2-[(4R)-4-(methoxycarbonyl)oxazoline]]cyclopropane. Yield: 42%. Gas chromatography area percentage: 97.6%.
¹H-NMR (300 MHz, CDCl₃, tetramethylsilane (TMS) reference) δ (ppm): 4.79 to 4.73 (2H, m), 4.55 to 4.43 (4H, m), 3.77 (6H, s), 1.55 (4H,d)
¹³C-NMR (75 MHz, CDCl₃, TMS reference)
δ (ppm): 171.31, 168.13, 69.93, 67.85, 52.55, 18.13, 16.68

### Example 1-3: Production example of compound (13) (n is 0)

A 200 mL round-bottomed four-necked flask equipped with a Dimroth condenser having a nitrogen introduction tube, a thermometer, a magnetic rotor and a dropping funnel was purged with nitrogen, then, into the flask were charged 2.00 g (6.53 mmol) of 1,1-bis[2-[(4R)-4-(methoxycarbonyl)oxazoline]]cyclopropane and 100 mL of dehydrated tetrahydrofuran and these were mixed, then, the mixture was cooled down to -66°C. Into the resultant mixture, 39.2 mL (39.2 mmol) of a methylmagnesium bromide/tetrahydrofuran solution having a concentration of 1.0 mol/L was dropped at -66°C over a period of 33 minutes. The resultant reaction mixture was gradually heated up to 0°C over a period of 3 hours, then, stirred at 0°C for 1 hour. The resultant reaction mixture was cooled down to -60°C, then, 40 mL of a saturated ammonium chloride aqueous solution was dropped, and further, 40 mL of water was dropped. The resultant mixture was heated up to room temperature, then, extracted with 200 mL of ethyl acetate five times. The resultant organic layers were mixed, washed with 100 mL of saturated saline, then, the resultant organic layer was dried over anhydrous sodium sulfate. Sodium sulfate was removed by filtration, then, the resultant filtrate was concentrated, and the resultant residue was purified by recrystallization from ethyl acetate/hexane = 6/4 twice, to give 889 mg of 1,1-bis[2-[(4R)-4-(2-hydroxy-2-propyl)oxazoline]]cyclopropa ne as a colorless crystal. Yield: 46%. Gas chromatography area percentage: 98.7%.
¹H-NMR (300 MHz, CDCl₃, tetramethylsilane (TMS) reference)
δ (ppm): 4.41 to 4.36 (2H, m), 4.21 to 4.15 (2H, m), 4.03 to 3.98 (2H, m), 3.42 (2H, s), 1.52 to 1.24 (4H, m), 1.30 (6H, s), 1.15 (6H, s)
¹³C-NMR (75 MHz, CDCl₃, TMS reference)
5 (ppm): 167.38, 74.92, 71.44, 69.07, 26.78, 25.44, 18.80, 13.66

### Example 1-4: Production example of compound (1) (n is 0 and R¹ to R³ are all an isopropyl group)

A 30 mL two-necked flask equipped with a nitrogen introduction tube and a magnetic rotor was purged with nitrogen, then, into the flask were charged 70.2 mg (1.76 mmol) of 60% sodium hydride and 5 mL of dehydrated tetrahydrofuran, and the mixture was cooled to 0°C. To the resultant mixture was added 100 mg (0.337 mmol) of 1,1-bis[2-[(4R)-4-(2-hydroxy-2-propyl)oxazoline]]cyclopropa ne, and the mixture was stirred at 0°C for 70 minutes. To the resultant mixture was added 342 mg (1.69 mmol) of triisopropylsilyl chloride at 0°C, then, the resultant mixture was heated up to room temperature, then, stirred at room temperature overnight, and further refluxed for 1 hour. The resultant reaction mixture was concentrated, and the resultant residue was purified by silica gel column chromatography (silica gel 12.5 g, hexane/ethyl acetate = 95/5 to 65/35), to give 149 mg of pale yellow oily 1,1-bis[2-[(4R)-4-[2-(triisopropylsilyl)oxy-2-propyl]oxazol ine]]cyclopropane. Yield: 73%. Gas chromatography area percentage: 99.2%.
¹H-NMR (300 MHz, CDCl₃, tetramethylsilane (TMS) reference)
δ (ppm): 4.45 to 4.40 (2H, m), 4.25 to 4.18 (2H, m), 4.06 to 4.00 (2H, m), 1.45 to 1.41 (2H, m), 1.34 (6H, s), 1.32 to 1.27 (2H, m), 1.12 (6H, s), 1.09 to 1.03 (42H, m)
¹³C-NMR (75 MHz, CDCl₃, TMS reference)
δ (ppm) : 166.42, 76. 01, 74.59, 69.50, 28.75, 24.17, 18.38, 18.28, 14.84, 13.24

### Example 1-5: Production example of compound (1) (n is 0, R¹ is a thexyl group and R² and R³ are methyl groups)

A 30 mL two-necked flask equipped with a nitrogen introduction tube and a magnetic rotor was purged with nitrogen, then, into the flask were charged 70.2 mg (1.76 mmol) of 60% sodium hydride and 5 mL of dehydrated tetrahydrofuran, and the mixture was cooled to 0°C. To the resultant mixture was added 100 mg (0.337 mmol) of 1,1-bis[2-[(4R)-4-(2-hydroxy-2-propyl)oxazoline]]cyclopropa ne, and the mixture was stirred at 0°C for 1 hour. To the resultant mixture was added 318 mg (1.69 mmol) of thexyldimethylsilyl chloride at 0°C, then, the resultant mixture was heated up to room temperature, then, stirred at room temperature overnight, and further, refluxed for 2.5 hours. The resultant reaction mixture was concentrated, and the resultant residue was purified by silica gel column chromatography (silica gel 12.5 g, hexane/ethyl acetate = 95/5 to 70/30), to give 78 mg of colorless oily 1,1-bis[2-[(4R)-4-[2-(thexyldimethylsilyl)oxy-2-propyl]oxaz oline]]cyclopropane.
Yield: 40%. Gas chromatography area percentage: 97.7%.
¹H-MMR (300 MHz, CDCl₃, tetramethylsilane (TMS) reference)
δ (ppm): 4.38 to 4.33 (2H, m), 4.22 to 4.16 (2H, m), 4.00 to 3.94 (2H, m), 1.64 to 1.55 (2H, m), 1.47 to 1.38 (2H, m), 1.35 to 1.26 (2H, m), 1.29 (6H, s), 1.12 (6H, s), 0.87 (6H, s), 0.85 (6H, s), 0.78 (12H, s), 0.13 (12H, s)
¹³C-NMR (75 MHz, CDCl₃, TMS reference)
δ (ppm): 166.35, 75.87, 75.13, 69.48, 34.06, 28.62, 24.78, 24.05, 20.24, 20.16, 18.60, 18.56, 18.38, 14.93, 0.14, 0.12

### Example 1-6: Production example of compound (1) (n is 0 and R¹ to R³ are all an ethyl group)

A 30 mL two-necked flask equipped with a nitrogen introduction tube and a magnetic rotor was purged with nitrogen, then, into the flask were charged 70.2 mg (1.76 mmol) of 60% sodium hydride and 5 mL of dehydrated tetrahydrofuran, and the mixture was cooled to 0°C. To the resultant mixture was added 100 mg (0.337 mmol) of 1,1-bis[2-[(4R)-4-(2-hydroxy-2-propyl)oxazoline]]cyclopropa ne, and the mixture was stirred at 0°C for 1.5 hours. To the resultant mixture was added 262 mg (1.69 mmol) of triethylsilyl chloride at 0°C, then, the resultant mixture was heated up to room temperature, then, stirred at room temperature overnight, and further, refluxed for 3 hours. The resultant reaction mixture was concentrated, and the resultant residue was purified by silica gel column chromatography (silica gel 12.5 g, hexane/ethyl acetate = 95/5 to 60/40), to give 63 mg of colorless oily 1,1-bis[2-[(4R)-4-[2-(triethylsilyl)oxy-2-propyl]oxazoline] ]cyclopropane. Yield: 36%. Gas chromatography area percentage: 98.2%.
¹H-NMR (300 MHz, CDCl₃, tetramethylsilane (TMS) reference)
δ (ppm): 4.39 to 4.34 (2H, m), 4.23 to 4.17 (2H, m), 3.98 to 3.93 (2H, m), 1.33 to 1.25 (2H, m), 1.27 (6H, s), 1.14 to 1.06 (2H, m), 1.10(6H, s), 0.97 to 0.90 (18H, m), 0.62 to 0.52 (12H, m)
¹³C-NMR (75 MHz, CDCl₃, TMS reference)
δ (ppm): 166.43, 75.66, 74.53, 69.42, 28.57, 24.32, 18.31, 14. 95, 6.98, 6.63

### Example 1-7: Production example of compound (1) (n is 0 and R¹ to R³ are all a phenyl group)

A 30 mL two-necked flask equipped with a nitrogen introduction tube and a magnetic rotor was purged with nitrogen, then, into the flask were charged 70.2 mg (1.76 mmol) of 60% sodium hydride and 5 mL of dehydrated tetrahydrofuran, and the mixture was cooled to 0°C. To the resultant mixture was added 100 mg (0.337 mmol) of 1,1-bis[2-[(4R)-4-(2-hydroxy-2-propyl)oxazoline]]cyclopropa ne, and the mixture was stirred at 0°C for 65 minutes. To the resultant mixture was added 524 mg (1.69 mmol) of triphenylsilyl chloride at 0°C, then, the resultant mixture was heated, and refluxed for 2 hours. The resultant reaction mixture was concentrated, and to the resultant residue was added 2 mL of methanol at room temperature. The resultant mixture was concentrated, and the resultant residue was purified by silica gel column chromatography (silica gel 12.5 g, hexane/ethyl acetate = 90/10 to 25/75), to give 243 mg of colorless oily 1,1-bis[2-[(4R)-4-[2-(triphenylsilyl)oxy-2-propyl]oxazoline ]]cyclopropane. Yield: 89%.
¹H-NMR (300 MHz, CDCl₃, tetramethylsilane (TMS) reference)
δ (ppm): 7.65 to 7.62 (12H, m), 7.42 to 7.31 (18H, m), 4.59 to 4.54 (2H, m), 4.23 to 4.17 (2H, m), 4.05 to 3.99 (2H, m), 1.46 to 1.39 (2H, m), 1.27 to 1.21 (2H, m), 1.17 (6H, s), 1.15(6H, s)
¹³C-NMR (75 MHz, CDCl₃, TMS reference)
δ (ppm): 166.62, 135.97, 135.41, 129.73, 127.71, 76.95, 75.64, 69.47, 28.37, 24.92, 18.39, 15.04

### Example 1-8: Production example of compound (1) (n is 0, R¹ is a tert-butyl group and R² and R³ are phenyl groups)

A 30 mL two-necked flask equipped with a nitrogen introduction tube and a magnetic rotor was purged with nitrogen, then, into the flask were charged 70.2 mg (1.76 mmol) of 60% sodium hydride and 5 mL of dehydrated tetrahydrofuran, and the mixture was cooled to 0°C. To the resultant mixture was added 100 mg (0.337 mmol) of 1,1-bis[2-[(4R)-4-(2-hydroxy-2-propyl)oxazoline]]cyclopropa ne, and the mixture was stirred at 0°C for 70 minutes. To the resultant mixture was added 488 mg (1.69 mmol) of tert-butyldiphenylsilyl chloride at 0°C, then, the resultant mixture was heated and refluxed for 2.5 hours. The resultant reaction mixture was concentrated, and the resultant residue was purified by silica gel column chromatography (silica gel 12.5 g, hexane/ethyl acetate = 95/5 to 50/50), to give 34 mg of pale yellow oily 1,1-bis[2-[(4R)-4-[2-(tert-butyldiphenylsilyl)oxy-2-propyl] oxazoline]]cyclopropane. Yield: 13%.
¹H-NMR (300 MHz, CDCl₃, tetramethylsilane (TMS) reference)
δ (ppm): 7.76 to 7.72 (8H, m), 7.47 to 7.35 (12H, m), 4.62 to 4.57 (2H, m), 4.33 to 4.27 (2H, m), 4.08 to 4.02 (2H, m), 1.52 to 1.48 (2H, m), 1.38 to 1.33 (2H, m), 1.09 (6H, s), 1.08 (6H, s), 1.01 (18H, s)
¹³C-NMR (75 MHz, CDCl₃, TMS reference)
δ (ppm): 167.22, 136. 85, 136.15, 136.06, 130. 30, 130.13, 128.22, 128.03, 76.99, 76.66, 70.20, 28.87, 27.81, 25.36, 20.11, 19.18, 15.64

### Example 1-9: Production example of compound (1) (n is 0, R¹ is a methyl group and R² and R³ are phenyl groups)

A 30 mL two-necked flask equipped with a nitrogen introduction tube and a magnetic rotor was purged with nitrogen, then, into the flask were charged 70.2 mg (1.76 mmol) of 60% sodium hydride and 5 mL of dehydrated tetrahydrofuran, and the mixture was cooled to 0°C. To the resultant mixture was added 100 mg (0.337 mmol) of 1,1-bis[2-[(4R)-4-(2-hydroxy-2-propyl)oxazoline]]cyclopropa ne, and the mixture was stirred at 0°C for 1 hour. To the resultant mixture was added 413 mg (1.69 mmol) of methyldiphenylsilyl chloride at 0°C, then, the resultant mixture was heated, and refluxed for 2 hours. To the resultant reaction mixture was added 1 mL of methanol at room temperature, and the mixture was stirred at room temperature for 10 minutes. The resultant mixture was concentrated, and the resultant residue was purified by silica gel column chromatography (silica gel 12.5 g, hexane/ethyl acetate = 95/5 to 50/50), to give 179 mg of pale yellow oily 1,1-bis[2-[(4R)-4-[2-(methyldiphenylsilyl)oxy-2-propyl]oxaz oline]]cyclopropane. Yield: 77%. Gas chromatography area percentage: 99.4%.
¹H-NMR (300 MHz, CDCl₃, tetramethylsilane (TMS) reference) 5 (ppm): 7.56 to 7.53 (8H, m), 7.40 to 7.30 (12H, m), 4.47 to 4.41 (2H, m), 4.21 to 4.14 (2H, m), 4.03 to 3.97 (2H, m), 1.44 to 1.35 (2H, m), 1.30 to 1.21 (2H, m), 1.23 (6H, s), 1.14 (6H, s), 0.68 (6H, s)
¹³C-NMR (75 MHz, CDCl₃, TMS reference)
δ (ppm) :166.54, 138.08, 138.05, 134.16, 134.13, 129.50, 129.43, 127.70, 127.66, 76.25, 75.56, 69.41, 28.47, 24.73, 18.35, 15.03, -0.03

### Example 2

Into a nitrogen-purged 50 mL Schlenk flask were charged 2.1 mg (0.0202 mmol) of copper(I) chloride, 14.0 mg (0.0222 mmol) of 1,1-bis[2-[(4R)-4-[2-(triisopropylsilyl)oxy-2-propyl]oxazol ine]]cyclopropane, 20.4 mg (0.0222mmol) of trityl tetrakis(pentafluorophenyl) borate and 6.92 g (54.0 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.33 g (20.2 mmol) of ethyl diazoacetate and 3.42 g (26.7 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 84% (ethyl diazoacetate reference)
Trans isomer/cis isomer ratio: 93/7
(The trans isomer of the compound denotes a compound in which an ethoxycarbonyl group at 1-position and an acetoxymethyl group at 2-position based on a cyclopropane ring plane exist on the opposite sides, and the cis isomer thereof denotes a compound in which an ester group at 1-position and an acetoxymethyl group at 2-position based on a cyclopropane ring plane exist on the same side.)
Optical purity: trans isomer 95% e.e. (+ form), cis isomer 44% e.e. (+ form)

### Comparative Example 1

Into a nitrogen-purged 50 mL Schlenk flask were charged 2.4 mg (0.0228 mmol) of copper (I) chloride, 7.68 mg (0.0253 mmol) of 1,1-bis[2-[(4S)-4-tert-butyloxazoline]]cyclopropane, 23.4 mg (0.0253 mmol) of trityl tetrakis(pentafluorophenyl) borate and 7.91 g (61.7 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.66 g (23.0 mmol) of ethyl diazoacetate and 3. 92 g (30.5 mmol) of 3-methyl-2-butenyl acetate at 21°C over a period of 4. 6 hours, and further, the mixture was reacted by stirring at 21°C for 30 minutes, to give a solution containing ethyl 3,3-dimethyl-2-(acetoxymethyl)cyclopropanecarboxylate.
Yield: 78% (ethyl diazoacetate reference)
Trans isomer/cis isomer ratio: 87/12
Optical purity: trans isomer 95% e.e. (+ form), cis isomer 25% e.e. (+ form).

### Example 3

Into a nitrogen-purged 50 mL Schlenk flask were charged 2.1 mg (0.0202 mmol) of copper(I) chloride, 13.3 mg (0.0222 mmol) of 1,1-bis[2-[(4R)-4-[2-(thexyldimethylsilyl)oxy-2-propyl]oxaz oline]]cyclopropane, 20.4 mg (0.0222 mmol) of trityl tetrakis(pentafluorophenyl) borate and 6.94 g (54.2 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.33 g (20.2 mmol) of ethyl diazoacetate and 3.42 g (26.7 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 78% (ethyl diazoacetate reference)
Trans isomer/cis isomer ratio: 90/10
Optical purity: trans isomer 95% e.e. (+ form), cis isomer 38% e.e. (+ form)

### Example 4

Into a nitrogen-purged 50 mL Schlenk flask were charged 2.2 mg (0.0211 mmol) of copper(I) chloride, 12.6 mg (0.0232 mmol) of 1,1-bis[2-[(4R)-4-[2-(triethylsilyl)oxy-2-propyl]oxazoline] ]cyclopropane, 21.4 mg (0.0232 mmol) of trityl tetrakis(pentafluorophenyl) borate and 7.25 g (56.6 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.43 g (21.1mmol) of ethyl diazoacetate and 3.58 g (27.9 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 82% (ethyl diazoacetate reference)
Trans isomer/cis isomer ratio: 91/9
Optical purity: trans isomer 94% e. e. (+ form), cis isomer 42% e.e. (+ form)

### Example 5

Into a nitrogen-purged 50 mL Schlenk flask were charged 2.6 mg (0.0250 mmol) of copper(I) chloride, 23.1 mg (0.0274 mmol) of 1,1-bis[2-[(4R)-4-[2-(triphenylsilyl)oxy-2-propyl]oxazoline ]]cyclopropane, 25.3 mg (0.0274 mmol) of trityl tetrakis(pentafluorophenyl) borate and 8.59 g (67.0 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.88 g (25.0 mmol) of ethyl diazoacetate and 4.24 g (33.1 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 53% (ethyl diazoacetate reference)
Trans isomer/cis isomer ratio: 91/9
Optical purity: trans isomer 95% e.e. (+ form), cis isomer 47% e.e. (+ form)

### Example 6

Into a nitrogen-purged 50 mL Schlenk flask were charged 2.1 mg (0.0202 mmol) of copper(I) chloride, 17.8 mg (0.0222 mmol) of 1,1-bis[2-[(4R)-4-[2-(tert-butyldiphenylsilyl)oxy-2-propyl] oxazoline]]cyclopropane, 20.5 mg (0.0222 mmol) of trityl tetrakis(pentafluorophenyl) borate and 6.95 g (54.2 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.33 g (20.2 mmol) of ethyl diazoacetate and 3.43 g (26.8 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 43% (ethyl diazoacetate reference)
Trans isomer/cis isomer ratio: 92/8
Optical purity: trans isomer 94% e.e. (+ form), cis isomer 35% e.e. (+ form)

### Example 7

Into a nitrogen-purged 50 mL Schlenk flask were charged 2.6 mg (0.0250 mmol) of copper(I) chloride, 19.6 mg (0.0274 mmol) of 1,1-bis[2-[(4R)-4-[2-(methyldiphenylsilyl)oxy-2-propyl]oxaz oline]]cyclopropane, 25.3 mg (0.0274 mmol) of trityl tetrakis(pentafluorophenyl) borate and 8.60 g (67.1 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.88 g (25.0 mmol) of ethyl diazoacetate and 4.24 g (33.1 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 47% (ethyl diazoacetate reference)
Trans isomer/cis isomer ratio: 89/11
Optical purity: trans isomer 97% e.e. (+ form), cis isomer 38% e.e. (+ form)

Comparison of Examples 2 to 7 and Comparative Example 1 described above teaches that selectivity of a trans isomer in an asymmetric cyclopropanation reaction is improved more in use of an asymmetric catalyst obtained from an optically active bisoxazoline compound of the present invention than in use of an asymmetric catalyst obtained from a conventionally known optically active bisoxazoline compound.

### Example 8

Into a nitrogen-purged 50 mL Schlenk flask were charged 2.4 mg (0.0231 mmol) of copper(I) chloride, 16.0 mg (0.0254 mmol) of 1,1-bis[2-[(4R)-4-[2-(triisopropylsilyl)oxy-2-propyl]oxazol ine]]cyclopropane, 28.1 mg (0.0254 mmol) of trityl tetrakis[3,5-bis(trifluoromethyl)phenyl] borate and 7.92 g (61.7 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.66 g (23.1 mmol) of ethyl diazoacetate and 3. 92 g (30.5 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 78% (ethyl diazoacetate reference)
Trans isomer/cis isomer ratio: 93/7
Optical purity: trans isomer 95% e.e. (+ form), cis isomer 44% e.e. (+ form)

### Example 9

Into a nitrogen-purged 50 mL Schlenk flask were charged 2.7 mg (0.0259 mmol) of copper(I) chloride, 18.0 mg (0.0285 mmol) of 1,1-bis[2-[(4R)-4-[2-(triisopropylsilyl)oxy-2-propyl]oxazol ine]]cyclopropane, 7.2 mg (0.0285 mmol) of silver hexafluorophosphate and 8.91 g (69.5 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.99 g (25.9 mmol) of ethyl diazoacetate and 4.40 g (34.3 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 82% (ethyl diazoacetate reference)
Trans isomer/cis isomer ratio: 87/13
Optical purity: trans isomer 91% e.e. (+ form), cis isomer 77% e.e. (+ form)

### Comparative Example 2

Into a nitrogen-purged 50 mL Schlenk flask were charged 2.2 mg (0.0212 mmol) of copper(I) chloride, 7.1 mg (0.0233 mmol) of 1,1-bis[2-[(4S)-4-tert-butyloxazoline]]cyclopropane, 5.9 mg (0.0233 mmol) of silver hexafluorophosphate and 7.27 g (56.7 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.44 g (21.2 mmol) of ethyl diazoacetate and 3.59 g (28.0 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 63% (ethyl diazoacetate reference)
Trans isomer/cis isomer ratio: 81/19
Optical purity: trans isomer 91% e.e. (+ form), cis isomer 68% e.e. (+ form).

Comparison of Example 9 and Comparative Example 2 described above teaches that selectivity of a trans isomer in an asymmetric cyclopropanation reaction is improved more in use of an asymmetric catalyst obtained from an optically active bisoxazoline compound of the present invention than in use of an asymmetric catalyst obtained from a conventionally known optically active bisoxazoline compound.

### Example 10

Into a nitrogen-purged 50 mL Schlenk flask were charged 2.2 mg (0.0212 mmol) of copper(I) chloride, 14.5 mg (0.0230 mmol) of 1,1-bis[2-[(4R)-4-[2-(triisopropylsilyl)oxy-2-propyl]oxazol ine]]cyclopropane, 3.5 mg (0.0230 mmol) of lithium hexafluorophosphate and 7.20 g (56.2 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.42 g (21.0 mmol) of ethyl diazoacetate and 3.56 g (27.8 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 58% (ethyl diazoacetate reference)
Trans isomer/cis isomer ratio: 86/14
Optical purity: trans isomer 91% e.e. (+ form), cis isomer 79% e.e. (+ form)

### Example 11

Into a nitrogen-purged 50 mL Schlenk flask were charged 2.3 mg (0.0219 mmol) of copper(I) chloride, 15.2 mg (0.0241 mmol) of 1,1-bis[2-[(4R)-4-[2-(triisopropylsilyl)oxy-2-propyl]oxazol ine]]cyclopropane, 8.3 mg (0.0241 mmol) of silver hexafluoroantimonate and 7.53 g (58.7 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.53 g (21.9 mmol) of ethyl diazoacetate and 3.72 g (29.0 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 78% (ethyl diazoacetate reference)
Trans isomer/cis isomer ratio: 88/12
Optical purity: trans isomer 92% e.e. (+ form), cis isomer 75% e.e. (+ form)

### Example 12

Into a nitrogen-purged 50 mL Schlenk flask were charged 5.8 mg (0.0109 mmol) of copper(I) trifluoromethanesulfonate-toluene complex, 15.1 mg (0.0239 mmol) of 1,1-bis[2-[(4R)-4-[2-(triisopropylsilyl)oxy-2-propyl]oxazol ine]]cyclopropane and 7.47 g (58.3 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.51 g (21.8 mmol) of ethyl diazoacetate and 3.70 g (28.8 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 76% (ethyl diazoacetate reference)
Trans isomer/cis isomer ratio: 86/14
Optical purity: trans isomer 92% e.e. (+ form), cis isomer 83% e.e. (+ form)

### Comparative Example 3

Into a nitrogen-purged 50 mL Schlenk flask were charged 5.1 mg (0.00935 mmol) of copper(I) trifluoromethanesulfonate-toluene complex, 6.2 mg (0.0206 mmol) of 1,1-bis[2-[(4S)-4-tert-butyloxazoline]]cyclopropane and 6.43 g (50.2 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 2.16 g (18.7 mmol) of ethyl diazoacetate and 3.17 g (24.8 mmol) 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 72% (ethyl diazoacetate reference)
Trans isomer/cis isomer ratio: 80/20
Optical purity: trans isomer 93% e.e. (+ form), cis isomer 80% e.e. (+ form)

Comparison of Example 12 and Comparative Example 3 described above teaches that selectivity of a trans isomer in an asymmetric cyclopropanation reaction is improved more in use of an asymmetric catalyst obtained from an optically active bisoxazoline compound of the present invention than in use of an asymmetric catalyst obtained from a conventionally known optically active bisoxazoline compound.

### Example 13

Into a nitrogen-purged 50 mL Schlenk flask were charged 2. 9 mg (0.0207 mmol) of copper(II) chloride, 14.4 mg (0.0228 mmol) of 1,1-bis[2-[(4R)-4-[2-(triisopropylsilyl)oxy-2-propyl]oxazol ine]]cyclopropane, 21.0 mg (0.0228 mmol) of trityl tetrakis(pentafluorophenyl) borate and 7.12 g (55.5 mmol) of 3-methyl-2-butenyl acetate at rcom temperature, the internal temperature was adjusted to 21°C and the mixture was stirred for 30 minutes, to give a colorless uniform solution containing an asymmetric catalyst. Thereafter, into the solution was dropped a mixed solution of 2.39 g (20.7 mmol) of ethyl diazoacetate and 3.52 g (27.5 mmol) of 3-methyl-2-butenyl acetate at 21°C over a period of 4.6 hours, and further, the mixture was reacted by stirring at 21°C for 30 minutes, to give a solution containing ethyl 3,3-dimethyl-2-(acetoxymethyl)cyclopropanecarboxylate.
Yield: 75% (ethyl diazoacetate reference)
Trans isomer/cis isomer ratio: 92/8
Optical purity: trans isomer 94% e.e. (+ form), cis isomer 5% e.e. (+ form)

### Example 14

Into a nitrogen-purged 50 mL Schlenk flask were charged 2.1 mg (0.0202 mmol) of copper(I) chloride, 14.0 mg (0.0222 mmol) of 1,1-bis[2-[(4R)-4-[2-(triisopropylsilyl)oxy-2-propyl]oxazol ine]]cyclopropane, 20.4 mg (0.0222 mmol) of trityl tetrakis(pentafluorophenyl) borate and 10.82 g (61.4 mmol) of 3-methyl-2-butenyl benzyl ether at room temperature, the internal temperature was adjusted to 21°C and the mixture was stirred for 30 minutes, to give a colorless uniform solution containing an asymmetric catalyst. Thereafter, into the solution was dropped a mixed solution of 2.33 g (20.2 mmol) of ethyl diazoacetate and 3.42 g (19.4 mmol) of 3-methyl-2-butenyl benzyl ether at 21°C over a period of 4.6 hours, and further, the mixture was reacted by stirring at 21°C for 30 minutes, to give a solution containing ethyl 3,3-dimethyl-2-(benzyloxymethyl)cyclopropanecarboxylate.
Yield: 86% (ethyl diazoacetate reference)
Trans isomer/cis isomer ratio: 95/5
(The trans isomer of the compound denotes a compound in which an ester group at 1-position and a benzyloxymethyl group at 2-position based on a cyclopropane ring plane exist on the opposite sides, and the cis isomer thereof denotes a compound in which an ester group at 1-position and a benzyloxymethyl group at 2-position based on a cyclopropane ring plane exist on the same side.)
Optical purity: trans isomer 94% e.e. (+ form), cis isomer 33% e.e. (+ form)
(The optical purity of the compound was determined by hydrolyzing an ester portion to generate a carboxylate, then, calculating the liquid chromatography area ratio.)

### Comparative Example 4

Into a nitrogen-purged 50 mL Schlenk flask were charged 2.3 mg (0.0221 mmol) of copper(I) chloride, 7.4 mg (0.0243 mmol) of 2,2-bis[2-[(4S)-4-tert-butyloxazoline]]cyclopropane, 22.4 mg (0.0243 mmol) of trityl tetrakis(pentafluorophenyl) borate and 11.86 g (67.3 mmol) of 3-methyl-2-butenyl benzyl ether at room temperature, the internal temperature was adjusted to 21°C and the mixture was stirred for 30 minutes, to give a colorless uniform solution containing an asymmetric catalyst. Thereafter, into the solution was dropped a mixed solution of 2.56 g (22.1 mmol) of ethyl diazoacetate and 3. 74 g (21.2 mmol) of 3-methyl-2-butenyl benzyl ether at 21°C over a period of 4.6 hours, and further, the mixture was reacted by stirring at 21°C for 30 minutes, to give a solution containing ethyl 3,3-dimethyl-2-(benzyloxymethyl)cyclopropanecarboxylate.
Yield: 89% (ethyl diazoacetate reference)
Trans isomer/cis isomer ratio: 93/7
Optical purity: trans isomer 96% e.e. (+ form), cis isomer 3% e.e. (- form)

Comparison of Example 14 and Comparative Example 4 described above teaches that selectivity of a trans isomer in an asymmetric cyclopropanation reaction is improved more in use of an asymmetric catalyst obtained from an optically active bisoxazoline compound of the present invention than in use of an asymmetric catalyst obtained from a conventionally known optically active bisoxazoline compound.

### Production Example 1-1

A 100 mL round-bottomed four-necked flask equipped with a Dimroth condenser having a nitrogen introduction tube, a thermometer, a magnetic rotor and a dropping funnel was purged with nitrogen, then, into the flask were charged 4.01 g (25.3 mmol) of D-serine methyl ester hydrochloride and 40 mL of dehydrated chloroform, and the mixture was cooled to 0°C. Into the resultant mixture, 5.40 g (53.4 mmol) of triethylamine was dropped at 0°C over a period of 30 minutes, then, a solution obtained by mixing 2.13 g (12.3 mmol) of dimethylmalonic acid dichloride and 8 mL of dehydrated chloroform was dropped at 0°C over a period of 1 hour. The resultant mixture was heated up to room temperature, and stirred overnight at room temperature. The resultant reaction mixture was purified by silica gel column chromatography (silica gel 125 g, hexane/ethyl acetate/tetrahydrofuran = 90/10/0 to 0/0/100), to give 4.07 g of colorless oily N,N'-bis[(1R)-(1-methoxycarbonyl)-2-hydroxyethyl]-1,3-propa nedicarboxamide. Yield: 99%.
¹H-NMR (300 MHz, DMSO-d6, tetramethylsilane (TMS) reference) δ (ppm): 7.75 (2H,d), 5.05 (2H,t), 4.37 to 4.31 (2H, m), 3.77 to 3.65 (4H, m), 3.63 (6H, s), 1.35 (6H, s)
¹³C-NMR (75 MHz, CDCl₃, TMS reference)
δ (ppm): 173.09, 170.81, 60.92, 55.13, 51.93, 49.03, 23.61

### Production Example 1-2

A 200 mL round-bottomed four-necked flask equipped with a Dimroth condenser having a nitrogen introduction tube, a thermometer and a magnetic rotor was purged with nitrogen, then, into the flask were charged 4.02 g (12.0 mmol) of N,N'-bis[(1R)-1-(methoxycarbonyl)-2-hydroxyethyl]-1,3-propa nedicarboxamide and 121 mL of dehydrated tetrahydrofuran, and the mixture was cooled to -10°C. To the resultant mixture was added 6.15 g (25.0 mmol) of a (methoxycarbonylsulfamoyl)triethylammonium hydroxide intramolecular salt at 0°C, then, heated up to room temperature, and stirred at room temperature for 10 minutes. The resultant mixture was heated and refluxed for 2.5 hours, then, cooled down to room temperature. The resultant reaction mixture was concentrated, and to the resultant residue were added 201 mL of chloroform and 20 mL of a 5 wt% sodium hydrogen carbonate aqueous solution, and these were mixed, then, the liquid was separated. The resultant organic layer was washed with 20 mL of saturated saline, then, the liquid was separated, and the resultant organic layer was dried over anhydrous sodium sulfate. Sodium sulfate was removed by filtration, then, the mixture was concentrated, and the resultant residue was purified by silica gel column chromatography (silica gel 228 g, hexane/ethyl acetate/tetrahydrofuran = 20/20/1 to 6/6/1), to give 2.18 g of pale yellow oily 2,2-bis[2-[(4R)-4-(methoxycarbonyl)oxazoline]]propane.
Yield: 61%. Gas chromatography area percentage: 96.8%.
¹H-NMR (300 MHz, CDCl₃, tetramethylsilane (TMS) reference)
δ (ppm) : 4.78 to 4.72 (2H, m), 4.53 to 4.39 (4H, m), 3.76 (6H, s), 1.53 (6H,d)
¹³C-NMR (75MHz, CDCl₃, TMS reference)
δ (ppm): 171.52, 171.28, 69.98, 67.87, 52.53, 38.76, 24.18

### Production Example 1-3

A 200 mL round-bottomed four-necked flask equipped with a Dimroth condenser having a nitrogen introduction tube, a thermometer, a magnetic rotor and a dropping funnel was purged with nitrogen, then, into the flask were charged 2.13 g (6.90 mmol) of 2,2-bis[2-[(4R)-4-(methoxycarbonyl)oxazoline]]propane and 107 mL of dehydrated tetrahydrofuran and these were mixed, then, cooled down to -68°C. Into this mixture, 41.4 mL (41.4 mmol) of a methylmagnesium bromide/tetrahydrofuran solution having a concentration of 1.0 mol/L was dropped at -68°C over a period of 30 minutes. The resultant reaction mixture was gradually heated up to 0°C over a period of 2 hours and 50 minutes, then, cooled down to -60°C. Into this reaction mixture, 42.6 mL of a saturated ammonium chloride aqueous solution was dropped, and further, 40 mL of water was dropped. The resultant mixture was heated up to room temperature, then, extracted with 200 mL of ethyl acetate five times. The resultant organic layers were mixed, washed with 100 mL of saturated saline, then, the resultant organic layer was dried over anhydrous sodium sulfate. Sodium sulfate was removed by filtration, then, the resultant filtrate was concentrated, and the resultant residue was purified by recrystallization from ethyl acetate/hexane = 2/1 three times, to give 415 mg of 2,2-bis[2-[(4R)-4-(2-hydroxy-2-propyl)oxazoline]]propane as a colorless crystal. Yield: 20%. Gas chromatography area percentage: 98.0%.
¹H-NMR (300 MHz, CDCl₃, tetramethylsilane (TMS) reference)
δ (ppm): 4.41 to 4.36 (2H, m), 4.26 to 4.20 (2H, m), 4.05 to 4.00 (2H, m), 3.09 (2H, s), 1.53 (6H, s), 1.27 (6H, s), 1.14 (6H, s)
¹³C-NMR (75 MHz, CDCl₃, TMS reference)
δ (ppm): 171.03, 74.45, 71.78, 69.40, 39.25, 26.41, 25.35, 23.46

### Production Example 1-4

A 30 mL two-necked eggplant-shaped flask equipped with a nitrogen introduction tube and a magnetic rotor was purged with nitrogen, then, into the flask were charged 68. 3 mg (1.71 mmol) of 60% sodium hydride and 5 mL of dehydrated tetrahydrofuran, and the mixture was cooled to 0°C. To the resultant mixture was added 100 mg (0.328 mmol) of 2,2-bis[2-[(4R)-4-(2-hydroxy-2-propyl)oxazoline]]propane, and the mixture was stirred at 0°C for 1 hour. To the resultant mixture was added 333 mg (1.64 mmol) of triisopropylsilyl chloride at 0°C, then, the resultant mixture was heated up to room temperature, and further, refluxed for 2 hours. To the resultant reaction mixture was added 1 mL of methanol at room temperature, and the mixture was stirred for 10 minutes at room temperature. The resultant reaction mixture was concentrated, and the resultant residue was purified by silica gel column chromatography (silica gel 12.5 g, hexane/ethyl acetate = 98/2 to 90/10), to give 137 mg of colorless oily 2,2-bis[2-[(4R)-4-[2-(triisopropylsilyl)oxy-2-propyl]oxazol ine]]propane. Yield: 68%. Gas chromatography area percentage: 99.32%.
¹H-NMR (300 MHz, CDCl₃, tetramethylsilane (TMS) reference) δ (ppm): 4.43 to 4.38 (2H, m), 4.22 to 4.16 (2H, m), 4.09 to 4.03 (2H, m), 1.48 (6H, s), 1.37 (6H, s), 1.13 (6H, s), 1.10 to 0.97 (42H, m)
¹³C-NMR (75 MHz, CDCl₃, TMS reference)
δ (ppm): 169.77, 76.08, 74.62, 69.41, 38.66, 28.79, 24.18, 24.10, 18.30, 13.25

### Reference Comparative Example 1

Into a nitrogen-purged 50 mL Schlenk flask were charged 2.8 mg (0.0269 mmol) of copper(I) chloride, 18.7 mg (0.0295 mmol) of 2,2-bis[2-[(4R)-4-[2-(triisopropylsilyl)oxy-2-propyl]oxazol ine]]propane, 27.2 mg (0.0295 mmol) of trityl tetrakis(pentafluorophenyl) borate and 9.24 g (72.1 mmol) of 3-methyl-2-butenyl acetate at room temperature, the internal temperature was adjusted to 21°C and the mixture was stirred for 30 minutes, to give a colorless uniform solution containing an asymmetric catalyst. Thereafter, into the solution was dropped a mixed solution of 3.10 g (26.9 mmol) of ethyl diazoacetate and 4.56 g (35.6 mmol) of 3-methyl-2-butenyl acetate at 21°C over a period of 4.6 hours, and further, the mixture was reacted by stirring at 21 °C for 30 minutes, to give a solution containing ethyl 3,3-dimethyl-2-(acetoxymethyl)cyclopropanecarboxylate.
Yield: 66% (ethyl diazoacetate reference)
Trans isomer/cis isomer ratio: 67/33
Optical purity: trans isomer 65% e.e. (+ form), cis isomer 6% e.e. (- form)

### Industrial Applicability

According to the process of the present invention, selectivity of a trans isomer in the resulting optically active cyclopropane compound is improved.

## Claims

1. An optically active bisoxazoline compound represented by formula (1): 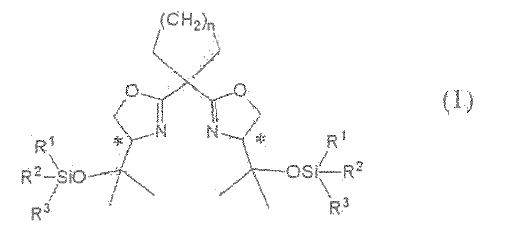 wherein R¹, R² and R³ each independently represent an alkyl group having 1 to 8 carbon atoms, a phenyl group or a benzyl group, n represents an integer of 0 to 3 and * represents an asymmetric center.

2. The compound according to Claim 1, wherein n is 0.

3. The compound according to Claim 1 or 2, wherein R¹, R² and R³ each independently represent an ethyl group or an isopropyl group.

4. The compound according to anyone of Claims 1 to 3, wherein R¹, R² and R³ are identical to each other.

5. The compound according to Claim 1 or 2, wherein R¹ is a thexyl group and R² and R³ are methyl groups.

6. An asymmetric catalyst obtained by mixing the following (A) and (B):
(A) a monovalent or divalent copper compound
(B) the compound according to any one of Claims 1 to 5.

7. The asymmetric catalyst according to Claim 6, obtained by mixing the (A) and the (B) and the following (C) or (D):
(C) a boron compound represented by formula (2): 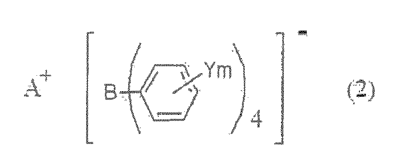 wherein A⁺ represents a lithium ion, a sodium ion, a potassium ion, a silver (I) ion or a trityl cation, Y represents a fluorine atom or a fluorinated alkyl group having 1 to 8 carbon atoms, m represents an integer of 1 to 5, and when m is 2 or more, the plurality of Y are the same or different
(D) a fluorine compound represented by formula (3):
D⁺ (MF₆)⁻ (3)
wherein D⁺ represents a lithium ion, a sodium ion, a potassium ion, a silver(I) ion or a trityl cation and M represents a phosphorus atom, an arsenic atom or an antimony atom.

8. The asymmetric catalyst according to Claim 6, obtained by mixing the (A), the (B) and the following (C):
(C) a boron compound represented by formula (2): 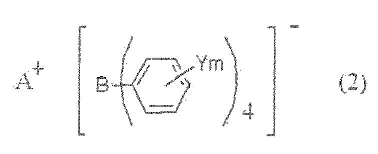 wherein A⁺ represents a trityl cation, Y represents a fluorine atom or a fluorinated alkyl group having 1 to 6 carbon atoms, m represents an integer of 1 to 5, and when m is 2 or more, the plurality of Y are the same or different.

9. A process for producing an optically active cyclopropane compound represented by formula (6): 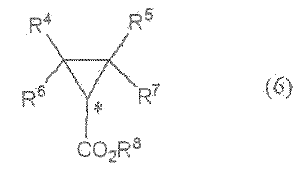 wherein R⁴, R⁵, R⁶, R⁷ and R⁸ are as defined below and * represents an asymmetric center,
the process comprising a step of reacting a prochiral olefin represented by formula (4): 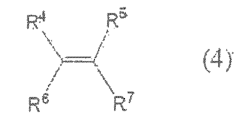 wherein R⁴, R⁵, R⁶ and R⁷ each independently represents a hydrogen atom, a halogen atom, an alkyl group having 1 to 6 carbon atoms and optionally having a substituent, an alkenyl group having 2 to 6 carbon atoms and optionally having a substituent, an aryl group having 6 to 10 carbon atoms and optionally having a substituent, an aralkyl group having 7 to 16 carbon atoms and optionally having a substituent or an alkoxycarbonyl group having 2 to 7 carbon atoms and optionally having a substituent, and R⁵ and R⁷ represent groups different from each other when R⁴ and R⁶ represent the same group,
with a diazoacetate ester represented by formula (5):
N₂CHCO₂R⁸ (5)
wherein R⁸ represents an alkyl group having 1 to 6 carbon atoms, in the presence of the asymmetric catalyst according to any one of Claims 6 to 8.

10. The process according to Claim 9, wherein R⁴ is a hydrogen atom or a fluorine atom, R⁵ is a methyl group, R⁶ is an acyloxymethyl group, an alkoxycarbonyloxymethyl group or an aralkyloxymethyl group and R⁷ is a methyl group.

11. The process according to Claim 10, wherein R⁶ is an acetoxymethyl group or a benzyloxymethyl group.

12. A process for producing an optically active bisoxazoline compound represented by formula (1): 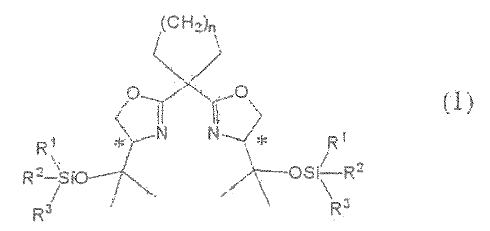 wherein R¹, R² and R³ each independently represent an alkyl group having 1 to 8 carbon atoms, a phenyl group or a benzyl group, n represents an integer of 0 to 3 and * represents an asymmetric center,
comprising the following first step, second step, third step and fourth step:
<First step>
a step of reacting an optically active serine ester represented by formula (7): 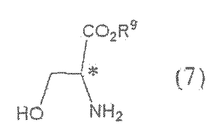 wherein R⁹ represents an alkyl group having 1 to 6 carbon atoms, a phenyl group or a benzyl group, and * represents an asymmetric center,
or a salt thereof with a cycloalkanedicarboxylic acid derivative represented by formula (8): 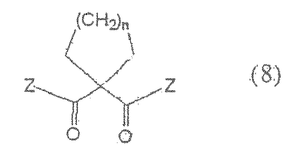 wherein Z represents a halogen atom or an alkoxy group having 1 to 6 carbon atoms, and n represents an integer of 0 to 3, to give an optically active diamide compound represented by formula (9): 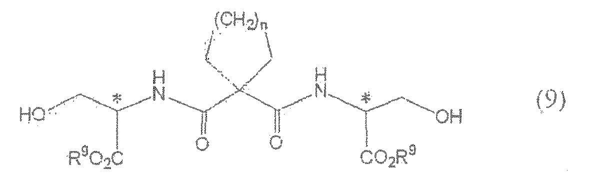 wherein R⁹, n and * are as defined above,
<Second step>
a step of reacting the optically active diamide compound represented by formula a (9) with a (methoxycarbonylsulfamoyl)triethylammonium hydroxide intramolecular salt to give an optically active bisoxazoline ester compound represented by formula (11): 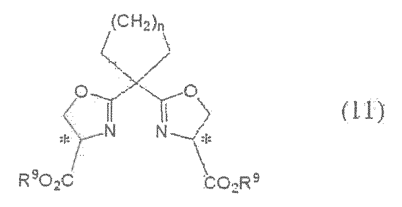 wherein R⁹, n and * are as defined above,
<Third step>
a step of reacting the optically active bisoxazoline ester compound represented by formula (11) with a Grignard reagent represented by formula (12):
CH₃MgQ (12)
wherein Q represents a halogen atom,
to give an optically active bisoxazoline alcohol compound represented by formula (13): 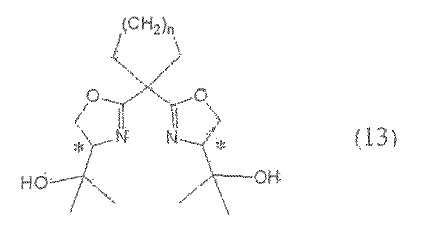 wherein, n and * are as defined above,
<Fourth step>
a step of reacting the optimally active bisoxazoline alcohol compound represented by formula (13) with a silicon compound represented by formula (14): 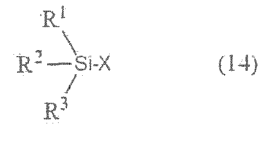 wherein R¹, R² and R³ are as defined above, and X represents a halogen atom,
to give the optically active bisoxazoline compound represented by formula (1).

13. An optically active diamide compound represented by formula (9): 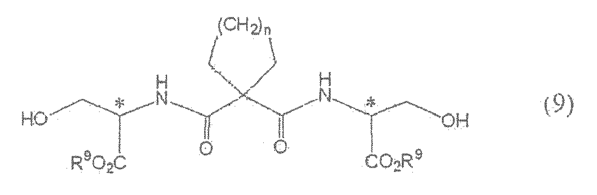 wherein R⁹ represents an alkyl group having 1 to 6 carbon atoms, a phenyl group or a benzyl group, n represents an integer of 0 to 3 and * represents an asymmetric center.

14. An optically active bisoxazoline ester compound represented by formula (11): 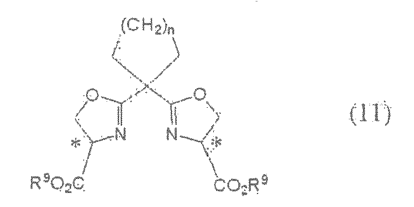 wherein R⁹ represents an alkyl group having 1 to 6 carbon atoms, a phenyl group or a benzyl group, n represents an integer of 0 to 3 and * represents an asymmetric center.

15. An optically active bisoxazoline alcohol compound represented by formula (13): 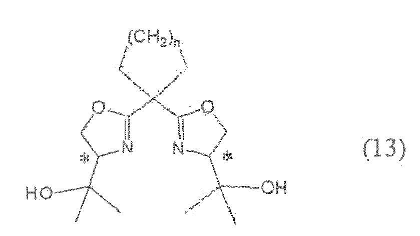 wherein n represents an integer of 0 to 3 and * represents an asymmetric center.
